# EUROPEAN PATENT APPLICATION

(11) **EP 2 241 330 A1**
(43) Date of publication of application: **20.10.2010**
(21) Application number: 10166138.7
(22) Date of filing: 13.02.2004
(51) Int. Cl.: A61K 39/395, A61K 38/00, A61P 15/18, C07K 16/40, G01N 33/68

(54) **Contraceptive methods and compositions related to proteasomal interference**

(30) Priority: 14.02.2003 US 447675 P
(62) Divisional of application: 04711198.4
(71) Applicant: The Curators Of The University Of Missouri, Columbia, MO 65211-2015 (US)
(72) Inventor: Sutovsky, Peter, Columbia, MO 65203 (US); Day, Billy N., Auxvasse, MO 65231 (US); McCauley, Tod C., Tucson, AZ 85750 (US); Sutovsky, Miriam, Columbia, MO 65203 (US)
(74) Representative: Dehmel, Albrecht

(57) **Abstract**

The present disclosure concerns the use of compositions and methods of regulating or evaluating fertility in an animal, in particular a mammal. In various embodiments of the disclosure, methods of contraception include inhibiting proteasome activity of a gamete, in particular spermatozoon. Proteasomal activity may be inhibited *in vitro* or *in vivo.* Inhibitors of the proteasome pathway include, but are not limited to small molecules, peptides, polypeptides (*e*.*g*., antibodies and the like) and affinity reagents that bind various components of the proteasome pathway (*e*.*g*., aptamers, *etc*.). In some embodiments, the activity of the proteasome pathway or the activity of a component in the proteasome pathway is inhibited by antibodies that bind and inhibit one or more components of the proteasome pathway.

SEQUENCE LISTING

<110> SUTOVSKY, PETER
      DAY, BILLY N.
      MCCAULEY, TOD C.
      SUTOVSKY, MIRIAM

<120> CONTRACEPTIVE METHODS AND COMPOSITIONS RELATED TO
      PROTEASOMAL INTERFERENCE

<130> FUL-200 EP DIV1

<140> not yet known
<141> 2010-06-16

<150> 60/447,675
<151> 2003-02-14

<160> 6

<170> PatentIn Ver. 2.1

<210> 1
<211> 30
<212> PRT
<213> Artificial Sequence

<220>
<223> Description of Artificial Sequence:  Synthetic
      Peptide

<400> 1
Asn Val Asp Ala Cys Val Ile Thr Ala Gly Gly Ala Lys Leu Gln Arg
  1               5                  10                  15

Ala Leu Ser Thr Pro Thr Glu Pro Val Gln Arg Ala Gly Arg
             20                  25                  30


<210> 2
<211> 19
<212> PRT
<213> Artificial Sequence

<220>
<223> Description of Artificial Sequence:  Synthetic
      Peptide

<400> 2
Thr Ala Gly Gly Ala Lys Leu Gln Arg Ala Leu Ser Thr Pro Thr Glu
  1               5                  10                  15

Pro Val Gln


<210> 3
<211> 645
<212> DNA
<213> Homo sapiens

<220>
<221> CDS

<222> (10)..(639)

<400> 3
cttgcaggg atg ctg cgc gcg gga gaa gtc cac acc ggg acc acc atc atg 51
Met Leu Arg Ala Gly Glu Val His Thr Gly Thr Thr Ile Met
1               5                   10

gca gtg gag ttt gac ggg ggc gtt gtg atg ggt tct gat tcc cga gtg 99
Ala Val Glu Phe Asp Gly Gly Val Val Met Gly Ser Asp Ser Arg Val
15                  20                  25                  30

tct gca ggc gag gcg gtg gtg aac cga gtg ttt gac aag ctg tcc ccg 147
Ser Ala Gly Glu Ala Val Val Asn Arg Val Phe Asp Lys Leu Ser Pro
                35                  40                  45

ctg cac gag cgc atc tac tgt gca ctc tct ggt tca gct gct gat gcc 195
Leu His Glu Arg Ile Tyr Cys Ala Leu Ser Gly Ser Ala Ala Asp Ala
            50                  55                  60

caa gcc gtg gcc gac atg gcc gcc tac cag ctg gag ctc cat ggg ata 243
Gln Ala Val Ala Asp Met Ala Ala Tyr Gln Leu Glu Leu His Gly Ile
        65                  70                  75

gaa ctg gag gaa cct cca ctt gtt ttg gct gct gca aat gtg gtg aga 291
Glu Leu Glu Glu Pro Pro Leu Val Leu Ala Ala Ala Asn Val Val Arg
    80                  85                  90

aat atc agc tat aaa tat cga gag gac ttg tct gca cat ctc atg gta 339
Asn Ile Ser Tyr Lys Tyr Arg Glu Asp Leu Ser Ala His Leu Met Val
95                  100                 105                 110

gct ggc tgg gac caa cgt gaa gga ggt cag gta tat gga acc ctg gga 387
Ala Gly Trp Asp Gln Arg Glu Gly Gly Gln Val Tyr Gly Thr Leu Gly
                115                 120                 125

gga atg ctg act cga cag cct ttt gcc att ggt ggc tcc ggc agc acc 435
Gly Met Leu Thr Arg Gln Pro Phe Ala Ile Gly Gly Ser Gly Ser Thr
            130                 135                 140

ttt atc tat ggt tat gtg gat gca gca tat aag cca ggc atg tct ccc 483
Phe Ile Tyr Gly Tyr Val Asp Ala Ala Tyr Lys Pro Gly Met Ser Pro
        145                 150                 155

gag gag tgc agg cgc ttc acc aca gac gct att gct ctg gcc atg agc 531
Glu Glu Cys Arg Arg Phe Thr Thr Asp Ala Ile Ala Leu Ala Met Ser
    160                 165                 170

cgg gat ggc tca agc ggg ggt gtc atc tac ctg gtc act att aca gct 579
Arg Asp Gly Ser Ser Gly Gly Val Ile Tyr Leu Val Thr Ile Thr Ala
175                 180                 185                 190

gcc ggt gtg gac cat cga gtc atc ttg ggc aat gaa ctg cca aaa ttc 627
Ala Gly Val Asp His Arg Val Ile Leu Gly Asn Glu Leu Pro Lys Phe
                195                 200                 205

tat gat gag tga accttc 645
Tyr Asp Glu
210

<210> 4
<211> 209
<212> PRT

<213> Homo sapiens

<400> 4
Met Leu Arg Ala Gly Glu Val His Thr Gly Thr Thr Ile Met Ala Val
1               5                   10                  15
Glu Phe Asp Gly Gly Val Val Met Gly Ser Asp Ser Arg Val Ser Ala
            20                  25                  30
Gly Glu Ala Val Val Asn Arg Val Phe Asp Lys Leu Ser Pro Leu His
        35                  40                  45
Glu Arg Ile Tyr Cys Ala Leu Ser Gly Ser Ala Ala Asp Ala Gln Ala
    50                  55                  60
Val Ala Asp Met Ala Ala Tyr Gln Leu Glu Leu His Gly Ile Glu Leu
65                  70                  75                  80
Glu Glu Pro Pro Leu Val Leu Ala Ala Ala Asn Val Val Arg Asn Ile
                85                  90                  95
Ser Tyr Lys Tyr Arg Glu Asp Leu Ser Ala His Leu Met Val Ala Gly
            100                 105                 110
Trp Asp Gln Arg Glu Gly Gly Gln Val Tyr Gly Thr Leu Gly Gly Met
        115                 120                 125
Leu Thr Arg Gln Pro Phe Ala Ile Gly Gly Ser Gly Ser Thr Phe Ile
    130                 135                 140
Tyr Gly Tyr Val Asp Ala Ala Tyr Lys Pro Gly Met Ser Pro Glu Glu
145                 150                 155                 160
Cys Arg Arg Phe Thr Thr Asp Ala Ile Ala Leu Ala Met Ser Arg Asp
                165                 170                 175
Gly Ser Ser Gly Gly Val Ile Tyr Leu Val Thr Ile Thr Ala Ala Gly
            180                 185                 190
Val Asp His Arg Val Ile Leu Gly Asn Glu Leu Pro Lys Phe Tyr Asp
        195                 200                 205
Glu

<210> 5
<211> 996
<212> DNA
<213> Homo sapiens

<220>
<221> CDS
<222> (89)..(910)

<400> 5
agcagaggac tttttagctg ctcactggcc ccgcttgtct gaccgactca tccgcccgcg 60

acccctaatc ccctctgcct gccccaag atg ctg aag cca gcc ctg gag ccc    112
                                Met Leu Lys Pro Ala Leu Glu Pro
                                1               5

cga ggg ggc ttc tcc ttc gag aac tgc caa aga aat gca tca ttg gaa    160
Arg Gly Gly Phe Ser Phe Glu Asn Cys Gln Arg Asn Ala Ser Leu Glu
    10                  15                  20

cgc gtc ctc ccg ggg ctc aag gtc cct cac gca cgc aag acc ggg acc    208
Arg Val Leu Pro Gly Leu Lys Val Pro His Ala Arg Lys Thr Gly Thr
25                  30                  35                  40

acc atc gcg ggc ctg gtg ttc caa gac ggg gtc att ctg ggc gcc gat    256
Thr Ile Ala Gly Leu Val Phe Gln Asp Gly Val Ile Leu Gly Ala Asp
                45                  50                  55

acg cga gcc act aac gat tcg gtc gtg gcg gac aag agc tgc gag aag    304
Thr Arg Ala Thr Asn Asp Ser Val Val Ala Asp Lys Ser Cys Glu Lys

60                  65                  70

atc cac ttc atc gcc ccc aaa atc tac tgc tgt ggg gct gga gta gcc     352
Ile His Phe Ile Ala Pro Lys Ile Tyr Cys Cys Gly Ala Gly Val Ala
        75                  80                  85

gcg gac gcc gag atg acc aca cgg atg gtg gcg tcc aag atg gag cta     400
Ala Asp Ala Glu Met Thr Thr Arg Met Val Ala Ser Lys Met Glu Leu
    90                  95                  100

cac gcg tta tct acg ggc cgc gag ccc cgc gtg gcc acg gtc act cgc     448
His Ala Leu Ser Thr Gly Arg Glu Pro Arg Val Ala Thr Val Thr Arg
105                 110                 115                 120

atc ctg cgc cag acg ctc ttc agg tac cag ggc cac gtg ggt gca tcg     496
Ile Leu Arg Gln Thr Leu Phe Arg Tyr Gln Gly His Val Gly Ala Ser
                125                 130                 135

ctg atc gtg ggc ggc gta gac ctg act gga ccg cag ctc tac ggc gtg     544
Leu Ile Val Gly Gly Val Asp Leu Thr Gly Pro Gln Leu Tyr Gly Val
            140                 145                 150

cat ccc cat ggc tcc tac agc cgt ctg ccc ttc aca gcc ctg ggc tct     592
His Pro His Gly Ser Tyr Ser Arg Leu Pro Phe Thr Ala Leu Gly Ser
        155                 160                 165

ggt cag gac gcg gcc ctg gcg gtg cta gaa gac cgg ttc cag ccg aac     640
Gly Gln Asp Ala Ala Leu Ala Val Leu Glu Asp Arg Phe Gln Pro Asn
    170                 175                 180

atg acg ctg gag gct gct cag ggg ctg ctg gtg gaa gcc gtc acc gcc     688
Met Thr Leu Glu Ala Ala Gln Gly Leu Leu Val Glu Ala Val Thr Ala
185                 190                 195                 200

ggg atc ttg ggt gac ctg ggc tcc ggg ggc aat gtg gac gca tgt gtg     736
Gly Ile Leu Gly Asp Leu Gly Ser Gly Gly Asn Val Asp Ala Cys Val
                205                 210                 215

atc aca aag act ggc gcc aag ctg ctg cgg aca ctg agc tca ccc aca     784
Ile Thr Lys Thr Gly Ala Lys Leu Leu Arg Thr Leu Ser Ser Pro Thr
            220                 225                 230

gag ccc gtg aag agg tct ggc cgc tac cac ttt gtg cct gga acc aca     832
Glu Pro Val Lys Arg Ser Gly Arg Tyr His Phe Val Pro Gly Thr Thr
        235                 240                 245

gct gtc ctg acc cag aca gtg aag cca cta acc ctg gag cta gtg gag     880
Ala Val Leu Thr Gln Thr Val Lys Pro Leu Thr Leu Glu Leu Val Glu
    250                 255                 260

gaa act gtg cag gct atg gag gtg gag taa gctgaggctt agagcttgga       930
Glu Thr Val Gln Ala Met Glu Val Glu
265                 270

acaaggggga ataaacccag aaaatacagt taaaaaaaaa aaaaacaaaa aaaaaaaaaa 990

aaaaaa                                                              996

<210> 6
<211> 273
<212> PRT
<213> Homo sapiens

<400> 6
Met Leu Lys Pro Ala Leu Glu Pro Arg Gly Gly Phe Ser Phe Glu Asn
1               5                   10                  15
Cys Gln Arg Asn Ala Ser Leu Glu Arg Val Leu Pro Gly Leu Lys Val
            20                  25                  30
Pro His Ala Arg Lys Thr Gly Thr Thr Ile Ala Gly Leu Val Phe Gln
        35                  40                  45
Asp Gly Val Ile Leu Gly Ala Asp Thr Arg Ala Thr Asn Asp Ser Val
    50                  55                  60
Val Ala Asp Lys Ser Cys Glu Lys Ile His Phe Ile Ala Pro Lys Ile
65                  70                  75                  80
Tyr Cys Cys Gly Ala Gly Val Ala Ala Asp Ala Glu Met Thr Thr Arg
                85                  90                  95
Met Val Ala Ser Lys Met Glu Leu His Ala Leu Ser Thr Gly Arg Glu
            100                 105                 110
Pro Arg Val Ala Thr Val Thr Arg Ile Leu Arg Gln Thr Leu Phe Arg
        115                 120                 125
Tyr Gln Gly His Val Gly Ala Ser Leu Ile Val Gly Gly Val Asp Leu
    130                 135                 140
Thr Gly Pro Gln Leu Tyr Gly Val His Pro His Gly Ser Tyr Ser Arg
145                 150                 155                 160
Leu Pro Phe Thr Ala Leu Gly Ser Gly Gln Asp Ala Ala Leu Ala Val
                165                 170                 175
Leu Glu Asp Arg Phe Gln Pro Asn Met Thr Leu Glu Ala Ala Gln Gly
            180                 185                 190
Leu Leu Val Glu Ala Val Thr Ala Gly Ile Leu Gly Asp Leu Gly Ser
        195                 200                 205
Gly Gly Asn Val Asp Ala Cys Val Ile Thr Lys Thr Gly Ala Lys Leu
    210                 215                 220
Leu Arg Thr Leu Ser Ser Pro Thr Glu Pro Val Lys Arg Ser Gly Arg
225                 230                 235                 240
Tyr His Phe Val Pro Gly Thr Thr Ala Val Leu Thr Gln Thr Val Lys
                245                 250                 255
Pro Leu Thr Leu Glu Leu Val Glu Glu Thr Val Gln Ala Met Glu Val
            260                 265                 270
Glu

## Description

### BACKGROUND OF THE INVENTION

### A. Field of the Invention

The present invention relates generally to the fields of reproductive biology, drug development, immunology and molecular biology. More particularly, it concerns methods and compositions related to inhibitors of the ubiquitin-proteasome pathway and antigens derived from components of the ubiquitin-proteasome pathway for regulation and evaluation of fertility or diagnosis of infertility.

### B. Description of Related Art

Ubiquitination is a widespread and evolutionarily conserved, yet highly substrate specific pathway of protein degradation in eukaryotic cells (Pickart, 1998; Laney and Hochstrasser, 1999). Postranslational protein modification by ubiquitin occurs via covalent attachment of a 76 amino acid ubiquitin residue to a lysine (Lys) residue within the substrates amino acid sequence via ubiquitin's C-terminal glycine (Gly) residue. Since the ubiquitin molecule carries seven Lys-residues of its own, this initial mono-ubiquitination is often followed by the Gly-Lys ligation of additional ubiquitin molecules to the first, substrate-bound ubiquitin monomer and triggers the formation of di-, tri-, tetra- or poly-ubiquitin chains (Hershko and Ciechanover, 1998). Mono-, di- and tri-ubiquitination serve a variety of purposes, including but not limited to lysosomal protein degradation, endocytosis of membrane receptors, signal transduction and transcriptional control (reviewed by Conaway *et al*., 2002; Glickman and Ciechanover, 2002). Tetra and poly-ubiquitination are consensus signals for protein docking and degradation by the 26-S proteasome, a multi-subunit protease with activity specific to ubiquitinated proteins (Tanaka and Tsurumi, 1997; Hochstrasser, 2002). Within the lumen of the barrel-shaped proteasome, the substrate protein is dissected into small peptides and released in the cytoplasm where the proteolysis is completed by a set of cytosolic endopeptidases (reviewed by Hochstrasser, 2002; Kohler *et al.,* 2001). The liberated poly-ubiquitin chains are not degraded by the proteasome, but are returned to the cytoplasm and reused for modification of other proteins (Chen *et al.,* 2002; Voges *et al.,* 1999).

Ubiquitination and proteasomal degradation of various substrates was documented in both the cytoplasm and the nucleus (Glickman and Ciechanover, 2002). Intriguingly, studies of mammalian reproduction yielded evidence that ubiquitination can also occur on the cell surface and in the extracellular space. Ubiquitin was detected in the ovarian follicular fluid (Einspanier *et al.,* 1993), seminal plasma (Lippert *et al.,* 1993), in the epididymal fluid (Hermo and Jacks, 2002; Sutovsky *et al.,* 2001b) and on the surface of defective spermatozoa passing through the epididymis (Sutovsky *et al.,* 2001a, b; 2002, 2003). Ubiquitination and proteasomal degradation of sperm receptor on the surface of egg viteline envelope occurs during ascidian fertilization (Sawada *et al.,* 2002a, b).

The ability of the mammalian spermatozoa to bind to and pass through the zona pellucida ("zona" or ZP) has been ascribed to a set of sperm surface receptors and proteolytic trypsin-like enzymes (reviewed by Primakoff and Myles, 2002), residing in the sperm acrosomal cap which undergoes exocytosis (Gerton, 2002) upon the binding to ZP3-sperm receptor of the ZP matrix (Wassarman and Litscher, 1995). Targeted mutations and knockouts of several candidate acrosomal proteins and sperm surface receptors did not eliminate the ability of mouse sperm to bind to and to penetrate/digest the ZP (reviewed by Talbot *et al.,* 2003; Evans, 2001). This suggests that a proteolytic system other than serine proteases exist in the sperm acrosome and contributes to the digestion of ZP during fertilization.

Current methods of contraception include surgical sterilization of women and oral contraceptive use by women, which are the most common methods of contraception in the U.S. Hormone modulating contraceptives include combined (estrogen/progestin) contraceptives, such as combined injectable contraceptives, combined oral contraceptives; and progestin-only contraceptives, such as norplant implants, progestin-only injectable contraceptives, or progestin-only pills. However, combined estrogen/progestin oral contraceptives may cause or lead to thromboembolic disorders, cerebrovascular accidents, coronary artery disease, liver abnormalities, estrogen dependent cancers, and pregnancy. Other methods of contraception include intrauterine devices; cervical cap; barrier methods, such as male condoms, female condoms, diaphragms, spermicides, or contraceptive sponge; and rhythm methods, which are highly dependent on the individuals involved. Many of these methods may have far reaching side effects both physiologically and physically. Thus, there is a need for improved methods of regulating fertility as well as methods for diagnosing fertility for both humans and a variety of domestic and wild animals.

### SUMMARY OF THE INVENTION

The present invention includes methods of regulating or evaluating fertility in a human being or in an animal, in particular a mammal, by inhibiting or detecting components of the ubiquitin-proteasome pathway. In various embodiments of the invention, contraceptive methods include inhibiting proteasomal activity of spermatozoa. As used herein, "contraceptive" means an agent that when administered deliberately prevents conception or pregnancy, either directly or indirectly. Similarly, as used herein, "contraception" means the deliberate prevention of conception or pregnancy, either directly or indirectly. For the purpose of this invention, an agent that inhibits the activity of a component of the proteasome pathway, including compositions that induce an immune response against a component of the proteasome pathway, may be considered a contraceptive. In some embodiments, the proteasomal activity of spermatozoa may be inhibited *in vitro, ex vivo* or *in vivo*. Inhibitors of the proteasome pathway include, but are not limited to, small molecules, peptides, polypeptides (*e.g*., antibodies and the like) and affinity reagents that bind various components of the ubiquitin-proteasome pathway (*e.g*., aptamers, *etc*.). In some embodiments, the activity of the ubiquitin-proteasome pathway or the activity of a component in the ubiquitin-proteasome pathway is inhibited by antibodies that bind and inhibit the activity of one or more polypeptides. A description of the various polypeptides associated with the ubiquitin-proteasome pathway may be found at the Online Mendelian Inheritance in Man (OMIM) database maintained by the National Center for Biotechnology Information (NCBI).

In certain embodiments, methods include the induction of antibodies by immunization of a subject or animal with at least one peptide or antigen derived from a protein component of the ubiquitin-proteasome pathway. The antigen may be a peptide derived from a proteasomal subunit, ubiquitin, ubiquitin-associated enzyme or polypeptide associated with the proteasomal pathway. In particular embodiments, a peptide is derived from an α-type and/or β-type subunit of the 20S proteasomal core, such as the MECL1 subunit, or from a subunit of the 19S regulatory cap or 11S activator complex present in certain types of proteasomes. One or more peptides or antigens of the invention may be comprised in a vaccine composition. A peptide of the invention can be a proteasome-derived peptide with an amino acid sequence, examples of which are set forth in SEQ ID NO: 1, 2, 4, and 6. The peptides or antigens of the invention can be in the context of a fusion protein. In some embodiments, a peptide or antigen of the invention may include multiple antigenic epitopes in a single polypeptide or composition, which may or may not be derived from various polypeptides. In certain embodiments, antibodies are induced by immunization with at least one polynucleotide encoding at least one peptide or antigen derived from a component of the proteasome pathway. The polynucleotide encoding at least one peptide or antigen can be comprised in an expression vector, such as a plasmid, linear expression element, or a viral expression vector. In particular embodiments, a peptide or antigen encoded by a polynucleotide may be derived from a proteasomal protein such as an α-type or β-type proteasomal subunit polypeptide.

In one embodiment of the invention, the activity of a proteasome is inhibited by contacting a spermatozoon or an oocyte or both with a proteasome inhibitor. The proteasome inhibitor can be comprised in a pharmaceutically acceptable formulation. In certain embodiments, a proteasome inhibitor or proteasome inhibitor composition includes MG132 and/or lactacystin. The pharmaceutically acceptable formulation can be formulated into solutions, suspensions, tablets, pills, capsules, sustained release formulations, powders, creams, ointments, salves, sprays, pumps, liposomes, suppositories, inhalants, patches or other delivery systems. A composition for inhibiting activity of the proteasome pathway may be administered by a route including, but not limited to intravenous, intraperitoneal, intradermal, intramuscular, intrauterine, dermal, nasal, buccal, vaginal, inhalation, or topical route of administration. In certain embodiments, the route of administration is intrauterine or vaginal.

In various embodiments of the invention, methods for inducing anti-proteasome antibodies in a mammal include administering to the mammal one or more peptides or antigens derived from a component of the proteasome pathway. Administering one or more peptides or antigens of the invention may induce production of an antibody that binds and inhibits the activity of a mammalian ubiquitin-proteasome pathway. In certain embodiments the mammal is a human, a farm animal, a domestic animal, a wild animal or a nuisance animal, *e.g*., rat, skunk, *etc*. Animal may include, but is not limited to pigs, cattle, horses, cats, rodents, rabbits, deer, raccoon, opossum, skunk and the like. One or more peptides or antigens can be administered by expression of a polynucleotide encoding one or more peptides or antigens. A polynucleotide may be administered by intramuscular, intravenous, subcutaneous, intraperitoneal, intradermal, oral, inhaled or other known routes of administration. One or more peptides or antigens may be administered by intramuscular, intravenous, subcutaneous, intraperitoneal, intradermal, oral, inhaled or other known routes of administration.

In some embodiments of the invention, methods include the diagnosis of infertility and/or evaluation/estimation of fertility by detecting the presence or absence of components of the ubiquitin-proteasome pathway associated with spermatozoa, oocytes or other reproductive tissues (e.g. testis, epididymis, ovary, prostate, seminal vesicle and other sex accessory glands), contributing to the fertility of an animal or a human. The methods may include, but are not limited to ELISA, protein blotting, immunohistochemistry and the like.

The invention further provides a method of *in vitro* fertilization comprising adding to a sperm sample used for said in vitro fertilization an inhibitor of proteasomal activity, wherein the inhibitor is added in a concentration insufficient to prevent fertilization but sufficient to prevent polyspermic fertilization. In the method, less than about 25% of fertilizations may be polyspermic, including less than 10% of fertilizations. The inhibitor may be added *in vitro* or *in vivo* by any method as is described herein. Any inhibitor may be used with the method as is also described herein.

It is contemplated that any method or composition described herein can be implemented with respect to any other method or composition described herein.

The use of the word "a" or "an" when used in conjunction with the term "comprising" in the claims and/or the specification may mean "one," but it is also consistent with the meaning of "one or more," "at least one," and "one or more than one."

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

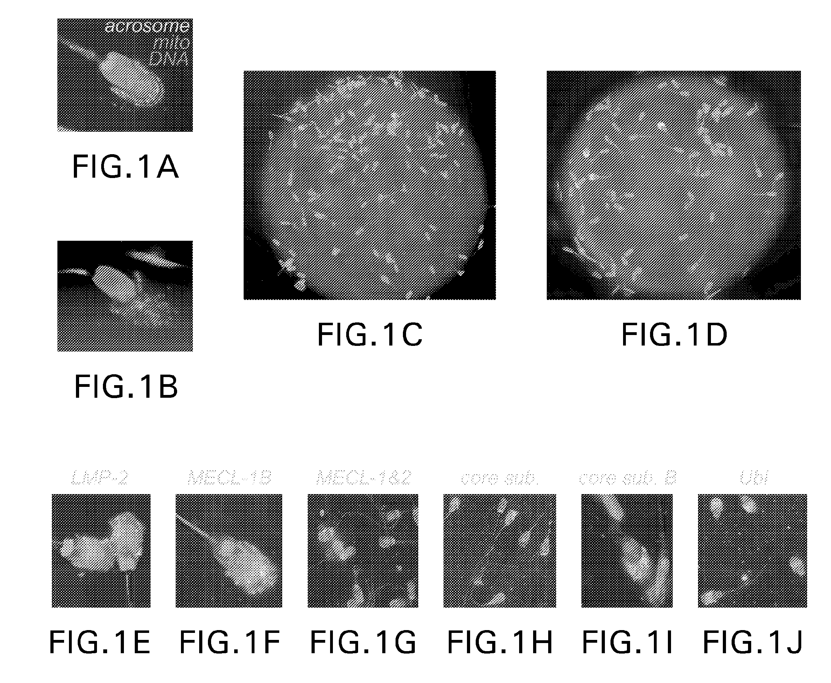

**FIG. 1A-1J** illustrates an example of sperm-ZP binding in proteasomal inhibitor-treated oocytes and the components of ubiquitin-proteasome system in porcine gametes. (FIG. 1A) Boar spermatozoon with intact acrosome labeled with antibody against resident tyrosine kinase c-Yes. (FIG. 1B) Remnants of c-Yes labeling after acrosome dispersion on the zona surface. (FIG. 1C) An oocyte fertilized in presence of 10 µM MG132 (a proteasomal inhibitor) shows only three spermatozoa with intact acrosomes at 20 hours post insemination (p.i.). This illustrates that while MG132 blocks fertilization in a highly specific fashion, it does not impair gamete viability, sperm-egg binding and sperm-acrosome reaction per se. (FIG. 1D) Control oocyte with three acrosome-intact spermatozoa at 20 hours p.i. Spermatozoa in FIG. 1A-D were pre-labeled with vital mitochondrial dye MitoTracker CMTM Ros. (FIG. 1E-I) Detection of boar sperm proteasomes by antibodies against subunits LMP-2, MECL 1 and MECL 2 and two different antibodies against proteasomal core subunits. (FIG. 1J) Ubiquitin detected by Ab 1690.

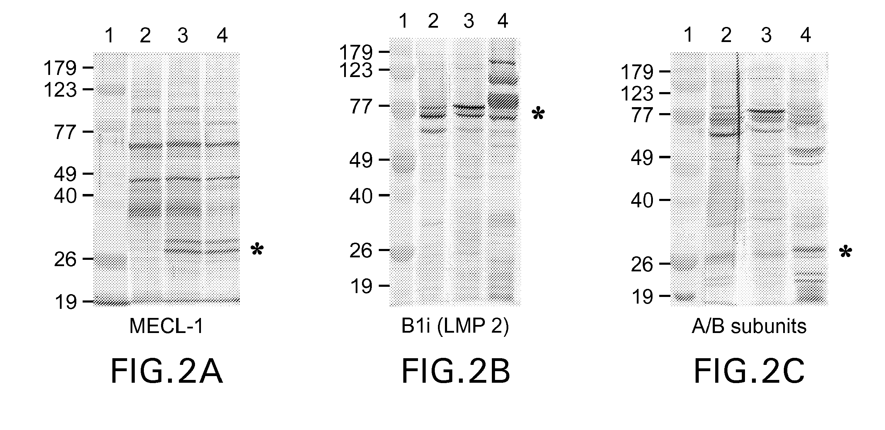

**FIG. 2A-2C** illustrates an example of immuno-detection of proteasomal subunits MECL-1 (FIG. 2A), β1i (FIG. 2B) and various α-type and β-type subunits (FIG. 2C) in the lysates of boar heat extracted (i.e. acrosome-less; lane 2), TX-100 extracted (lane 3) and intact (lane 4) sperm heads. Molecular weight markers are shown in lane 1.

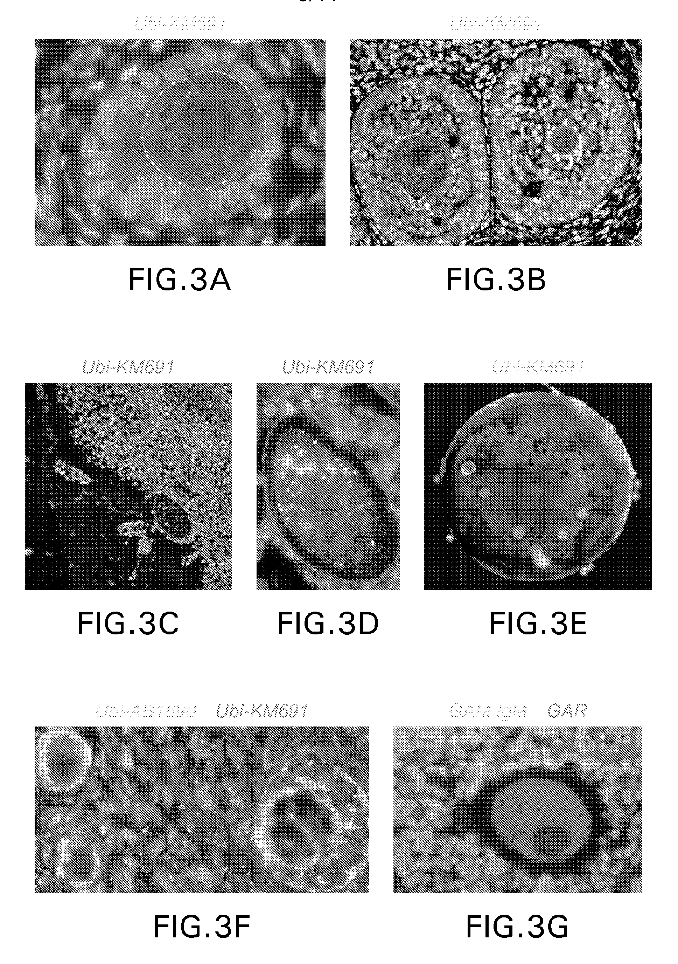

**FIG. 3A-3G** illustrates an example of ubiquitin immuno-reactive proteins on the outer face of porcine zona pellucida. Ubiquitin was detected by mouse monoclonal antibody KM 691 (FIG. 3A-D, FIG. 3F, FIG. 3E) or by rabbit serum AB1690 (FIG. 3E; FIG. 3F) inside preantral (FIG. 3A, FIG. 3B, FIG. 3F, FIG. 3G) and antral (FIG. 3C, FIG. 3D) porcine ovarian follicles, and on the surface of whole-mounted, full grown oocytes (FIG. 3E, germinal vesicle stage-oocyte is shown). Follicular granulosa cells in some of these paraffin sections (FIG. 3A, FIG. 3B) were counterstained with antibody against mitochondrial protein prohibitin. In FIG. 3E, labeling with Ab 1690 outlines nucleolus inside the germinal vesicle of this oocyte and shows that the labeling on the zona surface was not due to the impermeability of ZP to antibodies. Ubiquitin immuno-reactivity is not seen in a negative control tissue section treated by combining the omission of KM691 and incubation with preimmune rabbit serum, followed by appropriate fluorescently conjugate immunoglobulins (FIG. 3G). DNA in all images was counterstained with DAPI.

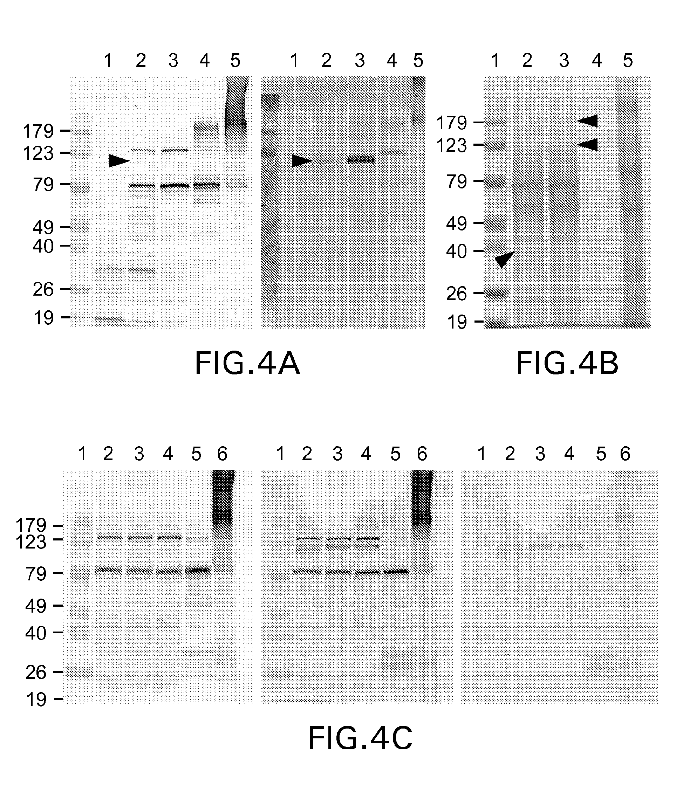

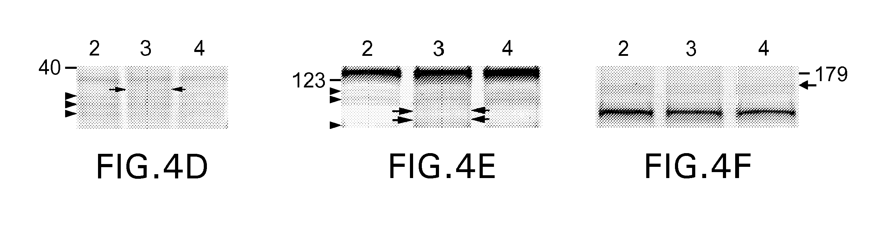

**FIG. 4A-4F** illustrates an example of immunodetection of the ubiquitinated substrates in lysates of the zona intact and zona-free porcine oocytes before fertilization and after fertilization with or without proteasomal inhibitor MG132. (FIG. 4A) Left panel: Lysates of the zona-free (lane 1) and zona-intact (lane 2), fertilized oocytes at 6 h post insemination, probed with anti-ubiquitin KM691 show a number of ubiquitin-immuno-reactive bands contributed by ZP. A set of lanes (arrowhead) migrating slightly below the 123 kDa marker are absent from the ZP+ /MG132- control oocytes, but not from the ZP+ MG132+ oocytes (lane 3). Such bands are not present in control lysates of human (lane 4) and boar (lane 5) spermatozoa. Right panel: Coomassie blue staining of the same oocyte gel after protein transfer shows that some of such <123 kDa bands (arrowhead) were not transferred completely from gels to membranes, and are absent from the ZP- oocyte-lane (lane 1) and reduced in the ZP+/MG132- lane (lane 2). Large amount of such proteins present is in the ZP+/MG132+ lane (lane 3). (FIG. 4B) Coomassie blue staining of gels prior to protein transfer. (FIG. 4C) Left panel: Proteins from ZP+ unfertilized oocytes (lane 2), ZP+/MG132- zygotes (lane 3), ZP+/MG132+ oocytes (lane 4) and human (lane 5) and boar (lane 6) spermatozoa, probed with anti-ubiquitin KM-691. Center: Overlap of the left panel with the gel stained after transfer with coomassie blue, as shown in the right panel.

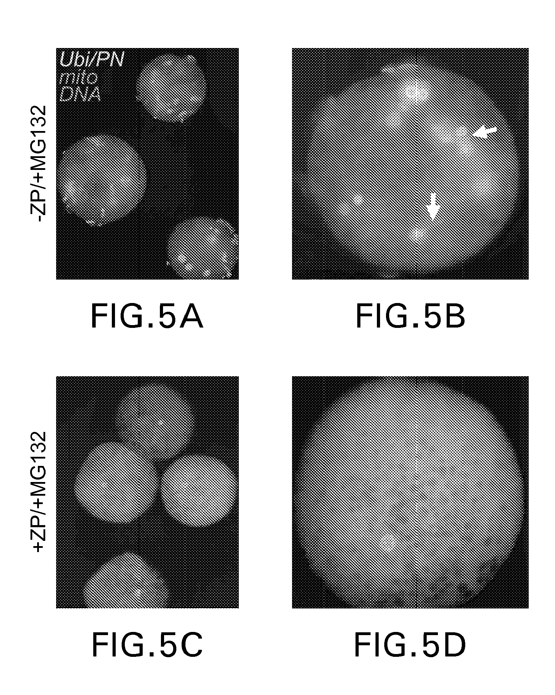

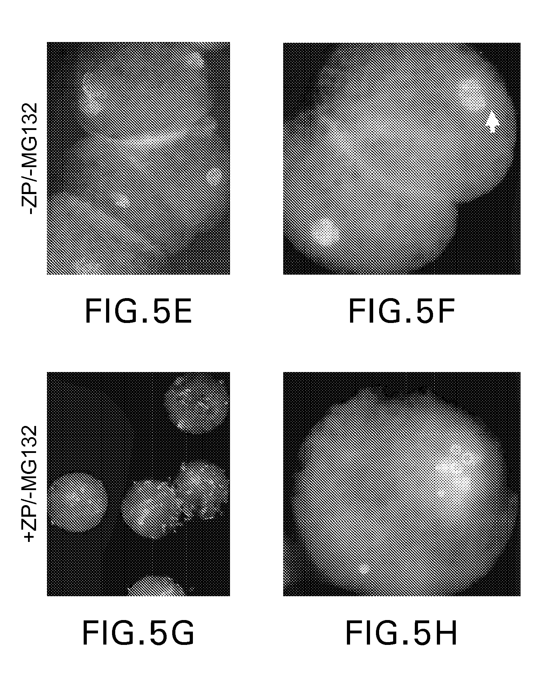

**FIG. 5A-5H** illustrates an example to the block of zona penetration by proteasomal inhibitors and the absence of such block in zona-free oocytes. MG132 inhibited in vitro fertilization in the zona-intact oocytes (FIG. 5C, FIG. 5D), but had no effect on the zona-free oocytes (FIG. 5A, FIG. 5B). Appropriate zona free (FIG. 5E, FIG. 5F) and zona intact (FIG. 5G, FIG. 5H) controls were performed. DNA was counterstained with DAPI, pronuclei and nucleoli inside the fertilized oocytes were labeled with antibody Ab 1690.

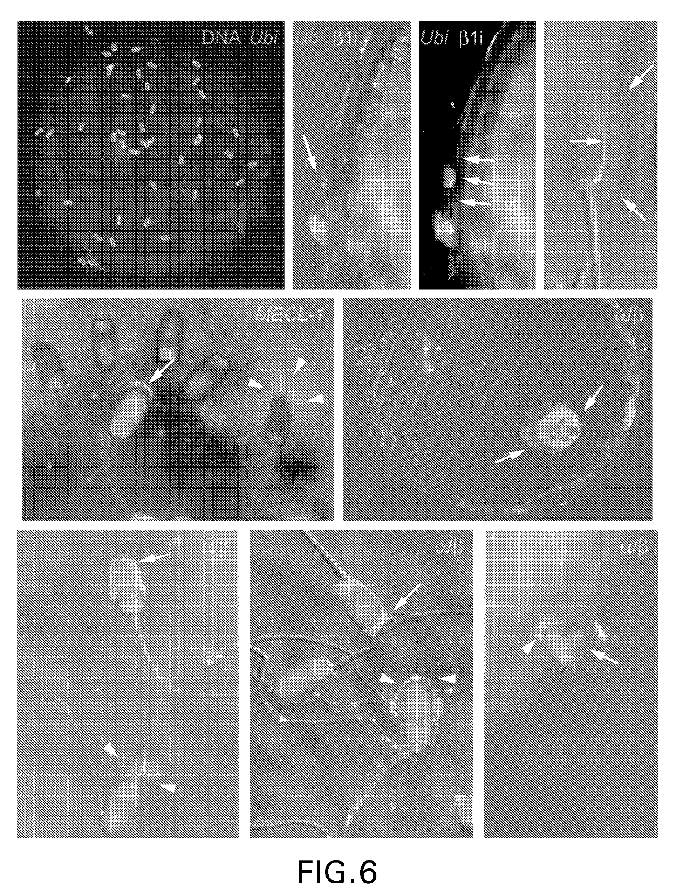

**FIG. 6** illustrates an example of interaction of sperm proteasomes with the ubiquitin immuno-reactive ZP proteins as visualized by anti-ubiquitin and anti-proteasome immunofluorescence combined with differential interference contrast microscopy (DIC) imaging of boar spermatozoa on the ZP surface 6 hours post insemination(p.i.).

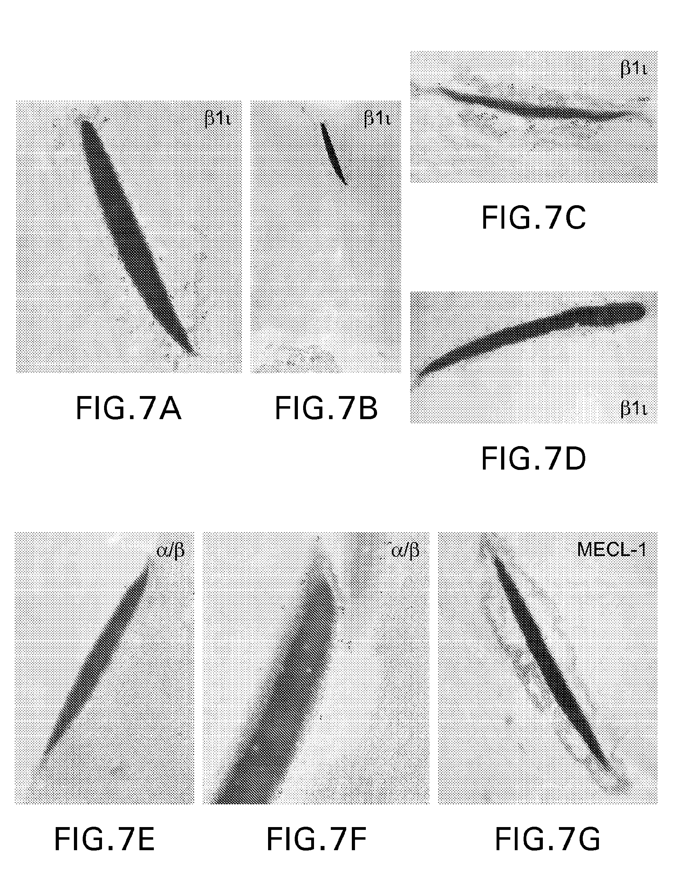

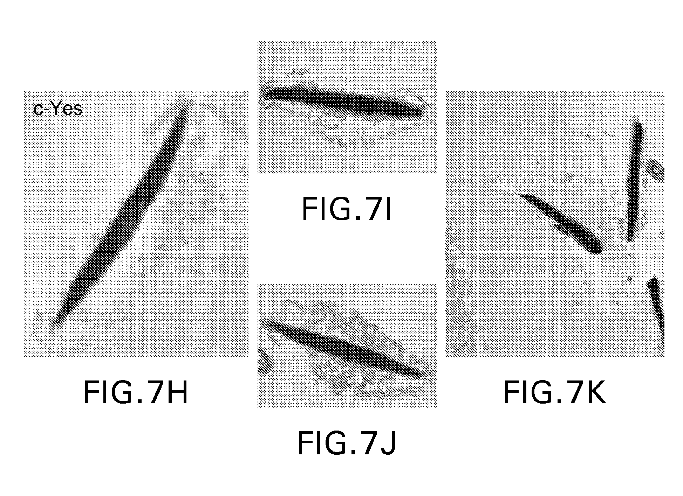

**FIG. 7A-7K** illustrates an example of immunoelectron-microscopic detection of proteasomal subunits during acrosome exocytosis. (FIG. 7A-G) Colloidal gold labeling of subunits β1i (FIG. 7A-D), α/β (FIG. 7E, F) and MECL-1 (FIG. 7G) is present in both acrosomal matrix and on the inner acrosomal membrane. (FIG. 7H) Acrosomal marker, tyrosine kinase c-yes is confined mainly to the complex of the outer acrosomal membrane and outer-acrosomal perinuclear theca. (FIG. 7I-K) Conventional electron microscopy shows the ultrastructure of acrosomal exocytosis during porcine fertilization.

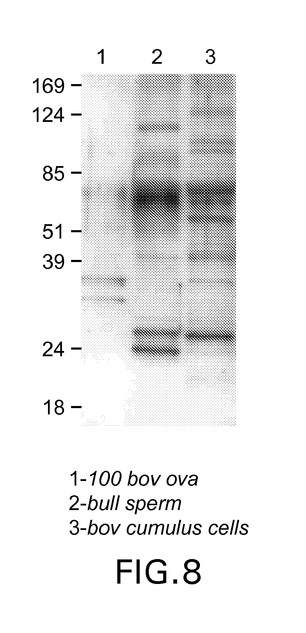

**FIG. 8**. Western blotting of proteasomal subunit MECL1 in 100 bovine ova (lane 1), bull sperm extracts (lane 2) and bovine cumulus cells (lane 3). Asterisk denotes the expected 29 kDa lane. Higher MW lanes, commonly seen in cell lysates of various cell types (ref. Groettrup *et al.,* 1996) are a result of other proteasomal subunit aggregation in process of sample preparation. The band of ∼32 kDa corresponds to the unprocessed MECL1-precursor protein.

### DESCRIPTION OF ILLUSTRATIVE EMBODIMENTS

As described above, there is a need for improved methods of regulating fertilization, evaluating fertility or diagnosing infertility. Most efforts in regulating fertility have focused on the use of chemical or mechanical methods of inhibiting gamete development or localization/mobility. There is a need for improved compositions and methods for fertility regulation. The invention as described herein, describes fertility regulating and diagnosing compositions and related methods. The invention includes inhibitors or binding agents for components of the ubiquitin-proteasome pathway for the regulation of fertility. The invention takes advantage of the surprising observation that penetration of the zona pellucida of an oocyte involves the proteasome and its related pathways, and can be inhibited by anti-proteasomal antibodies and inhibitors. The invention provides, as described herein, an improved method of reducing or inhibiting fertility and in some instances polyspermy, both *in vivo* and *in vitro.*

The Inventors have identified the fertility regulating properties of proteasomal inhibitors, in particular the compounds lactacystin and MG132, as well as antibodies that bind to and inhibit proteasome activity or block ubiquitin-dependent protein degradation. The inhibition of the mammalian ubiquitin-proteasome pathway blocks zona pellucida (ZP) penetration by spermatozoa. The inventors observed these new properties during successful attempts to reversibly block the degradation of the paternal mitochondria inside the zygotic cytoplasm. For paternal mitochondria degradation to occur, proteasomal inhibitors had to be added to fertilization medium only after the spermatozoa passed through the ZP and entered oocyte cytoplasm. The observation was unexpected due to the fact that ZP penetration was believed to be associated with serine protease activity and proteasomal inhibitors do not block the activity of serine proteases (Fenteany *et al.,* 1995; Goldberg *et al.,* 1995). In addition, the inventors have shown that besides ubiquitin and proteasomal subunits, the mammalian sperm acrosome contains the ubiquitin carrier E2, a conjugating enzyme required for the ubiquitin-substrate ligation, and possibly other deubiquitinating, ubiquitin-conjugating and ubiquitin-activating enzymes.

The invention, as disclosed herein, demonstrates that mammalian fertilization and ZP*-penetration rely on the proteolytic activity of sperm acrosomal proteasomes. In contrast to ascidians, where the spermatozoa seem to actively ubiquitinate the egg surface, ubiquitinated proteins may be acquired by the mammalian egg ZP prior to fertilization, or during oogenesis. Such data furnishes an important piece in the puzzle of mammalian fertilization as well as new contraceptive targets and infertility markers.

### I. UBIQUITIN-PROTEASOME PATHWAY

Compositions of the invention can be used as contraceptives or in contraceptive methods, as well as in diagnosing fertility. The inhibition or detection of polypeptides associated with the ubiquitin-proteasome pathway may be used to exert contraception or detect gametes with intact/defective or present/missing proteasome components, respectively.

The proteasome is widely recognized as the central enzyme complex of non-lysosomal protein degradation being an essential component of the ATP-dependent proteolytic pathway. The pathway catalyzes the rapid degradation of many rate-limiting enzymes, transcriptional regulators and critical regulatory proteins. The pathway is essential for the rapid elimination of highly abnormal proteins, arising via mutation or by post-translational damage, and plays a primary role in the slower degradation of the bulk of proteins in mammalian cells. It is also critically involved in higher eukaryotes in antigen processing.

The 26S proteasome is the key enzyme complex of the ubiquitin/ATP-dependent pathway of protein degradation. The catalytic core is formed by the 20S proteasome, a 2000 kDa complex, that is a barrel shaped structure, as shown by electron microscopy. The catalytic core is composed of four rings each containing seven subunits (subunits α1-7, β1-7).

Based on sequence similarity, all 20S proteasomal subunit sequences may be classified into two groups, α and β, each group having distinct structural and functional roles. The seven α-subunits comprise the outer rings and the seven β-subunits the inner rings of the 20S proteasome. Each subunit is located in a unique position within the α- or β-rings.

The typical 26S complex contains the 20S complex and over twenty additional proteins, ranging in molecular weight from 25 to 10kDa, located in a distinct complex called the 19S regulatory complex. The 26S complex determines substrate specificity and provides the multiple enzymatic functions necessary for proteolysis and viability. Systematic analysis of the sub-unit components have revealed at least six members to be ATPases belonging to a new family of ATP-binding proteins, together with a further fifteen sub-units that lack the capacity to bind ATP, isopeptidases and several other proteins thought to be responsible for the unfolding of a protein substrate prior to insertion into the proteolytic core of the 20S proteasome. In some proteasomes, the 19S regulatory complex is replaced by an 11S activator complex.

The 26S complex binds ATP and is responsible for the degradation of proteins that have been targeted for degradation by conjugation with ubiquitin. Ubiquitin, as described above, is attached to a target protein by an isopeptide bond formed between the epsilon-amino group of lysine on the target and the C-terminal glycine residue of ubiquitin by a series of ubiquitin activating (UBA) and conjugating (UBC) enzymes, e.g. enzymes E1, E2 and E3. Ubiquitin activating and conjugating enzymes act in series by transferring a ubiquitin molecule from one enzyme to the next, followed by the transfer of the activated ubiquitin molecule from the E2 enzyme to the target protein. The mono-ubiquitinylated protein is then acted upon again and the same enzymes attach an additional ubiquitin molecule to the previous one. Ubiquitin conjugation continues resulting in a high molecular weight protein complex. This poly-ubiquitinylated product then becomes the target for rapid degradation by the 26S proteasome with concomitant recycling of ubiquitin catalysed by the isopeptidases.

Although originally described as a complex with multiple peptidase activities, subsequent work has defined five activities for the 20S proteasome: a chymotrypsin-like activity that cleaves after large hydrophobic residues; a trypsin-like activity that cleaves after basic residues; a post-glutamyl hydrolase that cleaves after acidic residues; one which cleaves preferentially after branched-chain amino acids; and one cleaving after small neutral amino acids.

A second activator which can associate with the 20S proteasome in the absence of ATP is known as the 11S regulator. The pure PA28 activator is a complex of two alternating subunits, PA28α and PA28β, which share approximately 50% homology. Electron microscopic studies have shown PA28 to be a ring shaped particle which, like the 19S complex, caps the 20S proteasome, by binding to the alpha-rings, at both or either ends. The finding that PA28 modulates the proteasome-catalyzed production of antigenic peptides presented to the immune system on MHC class I molecules indicates a cellular function of this activator in antigen processing. For a detailed review see Schmid and Briand, 1997; Bogyo *et al.,* 1997.

In certain embodiments, various polypeptides associated with the ubiquitin-proteasome pathway, and fragments thereof, may be used. For example the ubiquitin-proteasome pathway associated polypeptides may include, but are not limited to components identified in the Online Mendelian Inheritance in Man (OMIM) database, examples of which include OMIM Nos. 600307 Proteasome Subunit, Beta-Type, 6; Psmb6 (Genbank Accession No. NM_002798); 176844 Proteasome Subunit, Alpha-Type, 5; Psma5(Genbank Accession No. NM_002790); 177045 Proteasome Subunit, Beta-Type, 9; Psmb9(Genbank Accession No. NM_002800 and NM_148954); 604030 Proteasome Subunit, Beta-Type, 7; Psmb7(Genbank Accession No. NM_002799); 600654 Proteasome Activator Subunit 1; Psme1(Genbank Accession No. NM_006263); 606223 Proteasome 26S Subunit, Non-ATPase, 2; Psmd2 (Genbank Accession No. NM_002808); 176847 Proteasome Subunit, Beta-Type, 10; Psmb10 (Genbank Accession No. NM_002801); 177046 Proteasome Subunit, Beta-Type, 8; Psmb8 (Genbank Accession No. NM_004159 and NM_148919); 602855 Proteasome Subunit, Alpha-Type, 6; Psma6 (Genbank Accession No. NM_002791); 602854 Proteasome Subunit, Alpha-Type, 1; Psma1 (Genbank Accession No. NM_002786 and NM_148976); 602161 Proteasome Activator Subunit 2; Psme2 (Genbank Accession No. NM_002818); 600306 Proteasome Subunit, Beta-Type, 5; Psmb5 (Genbank Accession No. NM_002797); 605129 Proteasome Activator Subunit 3; Psme3 (Genbank Accession No. NM_005789); 602175 Proteasome Subunit, Beta-Type, 2; Psmb2 (Genbank Accession No. NM_002794); 603146 Proteasome 26s Subunit, Non-ATPase, 9; Psmd9 (Genbank Accession No. NM_002813); 154365 Proteasome 26s Subunit, ATPase, 2; Psmc2 (Genbank Accession No. NM_002803); 604449 Proteasome 26s Subunit, Non-ATPase, 11; Psmdl11 (Genbank Accession No. NM_002815); 606607 Proteasome Subunit, Alpha-Type, 7; Psma7 (Genbank Accession No. NM_002792 and NM_152255); 176843 Proteasome Subunit, Alpha-Type, 3; Psma3 (Genbank Accession No. NM_002788 and NM_152132); 607173 Pad1; (Genbank Accession No. NM_005805); 604450 Proteasome 26s Subunit, Non-ATPase, 12; Psmd12 (Genbank Accession No. NM_002816); 602706 Proteasome 26s Subunit, ATPase, 1; Psmc1 (Genbank Accession No. NM_002802); 602708 Proteasome 26s Subunit, ATPase, 6; Psmc6 (Genbank Accession No. NM_002806); 186852 Proteasome 26s Subunit, ATPase, 3; Psmc3 (Genbank Accession No. NM_002804); 176842 Proteasome Subunit, Alpha-Type, 2; Psma2 (Genbank Accession No. NM_002787); 603481 Proteasome 26s Subunit, Non-ATPase, 13; Psmd13 (Genbank Accession No. NM_002817); 603480 Proteasome 26s Subunit, Non-ATPase, 10; Psmd10 (Genbank Accession No. NM_002814); 602017 Proteasome Subunit, Beta-Type, 1; Psmb1 (Genbank Accession No. NM_002793); 604452 Proteasome 26s Subunit, Non-ATPase, 5; Psmd5 (Genbank Accession No. NM_005047); 601648 Proteasome 26s Subunit, Non-ATPase, 4; Psmd4 (Genbank Accession No. NM_002810 and NM_153822); 602177 Proteasome Subunit, Beta-Type, 4; Psmb4 (Genbank Accession No. NM_002796); 602176 Proteasome Subunit, Beta-Type, 3; Psmb3 (Genbank Accession No. NM_002795); 602707 Proteasome 26s Subunit, ATPase, 4; Psmc4 (Genbank Accession No. NM_153001 and NM 006503); 601681 Proteasome 26s Subunit, ATPase, 5; Psmc5 (Genbank Accession No. NM_002805); 157970 Proteasome 26s Subunit, Non-ATPase, 7; Psmd7 (Genbank Accession No. NM_002811); 602163 Ubiquitin-Conjugating Enzyme E2e 2; Ube2e2 (Genbank Accession No. Z44894); 605046 Ubiquilin 1; Ubq1n1 (Genbank Accession No. NM_013438 and NM 053067); 602544 Parkin; Park2 (Genbank Accession No. NM_004562, NM_013987 and NM 013988); 314370 Ubiquitin-Activating Enzyme 1; Ube1 (Genbank Accession No. NM_003334 and NM_153280); 300264 Ubiquilin 2; Ubqln2 (Genbank Accession No. NM_013444); 180470 Ribophorin I; Rpn1 (Genbank Accession No. NM_002950); 191342 Ubiquitin Carboxyl-Terminal Esterase L1 (Genbank Accession No. NM_004181); 118888 Chymotrypsin-Like Protease; Ctrl (Genbank Accession No. NM_001907); 605532 Smad Ubiquitination Regulatory Factor 2 (Genbank Accession No. NM_022739); 607119 Double Ring Finger Protein (Genbank Accession No. NM_015435); 605624 Ariadne, Drosophila, Homolog Of, 1; Arih1 (Genbank Accession No. NM_005744); 605568 Smad Ubiquitination Regulatory Factor 1 (Genbank Accession No. AF199364); 603124 Ubiquitin-Conjugating Enzyme E2g 2; Ube2g2 (Genbank Accession No. NM_003343); 603091 Ubiquitin-Specific Protease 12; Usp12 (Genbank Accession No. AF022789); 602995 Ubiquitin-Conjugating Enzyme E2 Variant 1; Ube2v1 (Genbank Accession No. NM_003349, NM_021988 and NM_022442); 602961 Ubiquitin-Conjugating Enzyme E2d 1; Ube2d1 (Genbank Accession No. NM_003338); 602916 Ubiquitin-Conjugating Enzyme E2e 1; Ube2e1 (Genbank Accession No. NM_003341); and 300420 Praja 1; Pja1 (Genbank Accession No. NM_022368); each of which is ated herein by reference. Similar databases such as Genbank may also be used to identify ubiquitin-proteasome related polypeptides. Ubiquitin-proteasome related polypeptides refer to any polypeptide that is a part of the complex or pathway or influences the pathways activity or composition.

### II. POLYPEPTIDES AND PROTEINACEOUS COMPOSITIONS

In various aspects of the invention, proteinaceous compositions including peptides, polypeptides or proteins may be used as antigens, antibodies, vaccines, blockers, therapeutics or as components in compositions and methods described herein. The peptides, polypeptides and/or proteins may be an isolated/purified, a recombinant or a synthetic peptide(s), polypeptide(s) and/or protein(s).

Polypeptides and peptides of the invention may comprise immunogenic peptides, antigens or epitopes, or antibodies and the like directed to one or more protein components of a mammalian ubiquitin-proteasome and its related pathway. Such peptides may be useful for eliciting: an immune response against proteasomal proteins across various mammalian species, including, for example, rabbits, cows, pigs, horses, baboons, and humans. By eliciting an immune response against proteasomal proteins, the peptides of the invention are useful as contraceptive agents. In other embodiments, antibodies may be isolated and used in contraceptive or diagnostic compositions.

In certain embodiments, a proteasomal polypeptide or antigen may be a synthetic peptide. In still other embodiments, the peptide may be a recombinant peptide produced through molecular engineering techniques. The present section describes the methods and compositions involved in producing a proteinaceous composition for use in the present invention.

### A. Polypeptides

A polypeptide derived from one or more protein component of the ubiquitin-proteasome pathway, *i*.*e*., may be a naturally-occurring polypeptide that has been extracted using protein extraction techniques well known to those of skill in the art. In particular embodiments, a proteasomal antigen may be identified and prepared in a pharmaceutically acceptable carrier for vaccination of a mammal.

Sequence variants of the polypeptide may be prepared. Polypeptide sequence variants may be minor sequence variants of the polypeptide that arise due to natural variation within the population or they may be homologues found in other animals. They also may be sequences that do not occur naturally, but that are sufficiently similar that they function similarly and/or elicit an immune response that cross-reacts with natural forms of the polypeptide. Sequence variants can be prepared by standard methods of site-directed mutagenesis such as those described in Sambrook *et al.* 2001.

Another synthetic or recombinant variation of an antigenic proteasomal polypeptide is a polyepitope moiety comprising repeats of epitope determinants found naturally in proteasomal proteins. Such synthetic polyepitope proteins can be made up of several homomeric repeats of any one proteasomal protein epitope; or may comprise two or more epitopes from the same or different polypeptide.

Amino acid sequence variants of a polypeptide can be substitutional, insertional or deletion variants. Deletion variants lack one or more residues of the native protein which are not essential for function or immunogenic activity. Another common type of deletion variant is one lacking secretory signal sequences or signal sequences directing a protein to bind to a particular part of a cell.

Substitutional variants typically contain the exchange of one amino acid for another at one or more sites within the protein, and may be designed to modulate one or more properties of the polypeptide such as stability against proteolytic cleavage. Substitutions preferably are conservative, that is, one amino acid is replaced with one of similar shape and charge. Conservative substitutions are well known in the art and include, for example, the changes of: alanine to serine; arginine to lysine; asparagine to glutamine or histidine; aspartate to glutamate; cysteine to serine; glutamine to asparagine; glutamate to aspartate; glycine to proline; histidine to asparagine or glutamine; isoleucine to leucine or valine; leucine to valine or isoleucine; lysine to arginine; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine.

Insertional variants include fusion proteins such as those used to allow rapid purification of the polypeptide and also can include hybrid proteins containing sequences from other proteins and polypeptides that are homologues of the polypeptide. For example, an insertional variant could include portions of the amino acid sequence of the polypeptide from one species, together with portions of the homologous polypeptide from another species. The term "chimeric protein" is used to refer to a protein that is comprised of segments from two different proteins, be it homologous or non-homologous proteins. The segments need not be naturally adjacent segments nor is there a requirement for any segment(s) to maintain any particular location or order. Other insertional variants can include those in which additional amino acids are introduced within the coding sequence of the polypeptide. These typically are smaller insertions than the fusion proteins described above and are introduced, for example, into a protease cleavage site.

In one embodiment, major antigenic determinants of the polypeptide may be identified by an empirical approach in which portions of the gene encoding the polypeptide are expressed in a recombinant host, and the resulting proteins tested for their ability to elicit an immune response. For example, the polymerase chain reaction (PCR) can be used to prepare a range of cDNAs encoding peptides lacking successively longer fragments of the C-terminus of the protein. The immunogenic activity of each of these peptides then identifies those fragments or domains of the polypeptide that are essential for this activity. Further experiments in which only a small number of amino acids are removed or added at each iteration then allows the location of other antigenic determinants of the polypeptide. Thus, the polymerase chain reaction, a technique for amplifying a specific segment of DNA *via* multiple cycles of denaturation-renaturation, using a thermostable DNA polymerase, deoxyribonucleotides and primer sequences is contemplated in the present invention (Mullis, 1990; Mullis *et al.,* 1992).

Another embodiment for the preparation of the polypeptides according to the invention is the use of peptide mimetics. Mimetics are molecules that mimic elements of protein secondary structure. Because many proteins exert their biological activity *via* relatively small regions of their folded surfaces, their actions can be reproduced by much smaller designer (mimetic) molecules that retain the bioactive surfaces and have potentially improved pharmacokinetic/dynamic properties (Fairlie *et al.,* 1998). Methods for mimicking individual elements of secondary structure (helices, turns, strands, sheets) and for assembling their combinations into tertiary structures (helix bundles, multiple loops, helix-loop-helix motifs) have been reviewed (Fairlie *et al.,* 1998; Moore, 1994). Methods for predicting, preparing, modifying, and screening mimetic peptides are described in U.S. Patent 5,933,819 and U.S. Patent 5,869,451.

The following is a discussion based upon changing the amino acids of a protein or polypeptide to create an equivalent, or even an improved, second-generation molecule. The amino acid changes may be achieved by changing the codons of the DNA sequence, or by chemical peptide synthesis, according to the following examples.

For example, certain amino acids may be substituted for other amino acids in a polypeptide structure without appreciable loss of interactive binding capacity with structures such as, for example, antigen-binding regions of antibodies or binding sites on substrate molecules. Since it is the interactive capacity and nature of a polypeptide that defines the biological activity, certain amino acid substitutions can be made in a polypeptide sequence, and its underlying DNA coding sequence and nevertheless obtain a polypeptide with like or improved properties. Table 1 shows the codons that encode particular amino acids.

In making such changes, the hydropathic index of amino acids may be considered. The importance of the hydropathic amino acid index in conferring interactive biologic function on a protein is generally understood in the art (Kyte and Doolittle, 1982).

It is known in the art that certain amino acids may be substituted by other amino acids having a similar hydropathic index or score and still result in a protein or polypeptide with similar biological activity. It also is understood in the art that the substitution of like amino acids can be made effectively on the basis of hydrophilicity. U.S. Patent 4,554,101, ated here-in by reference, states that the greatest local average hydrophilicity of a protein, as governed by the hydrophilicity of its adjacent amino acids, correlates with a biological property of the protein.

Amino acid substitutions generally are based on the relative similarity of the amino acid side-chain substituents, for example, their hydrophobicity, hydrophilicity, charge, size, and the like. Exemplary substitutions that take various foregoing characteristics into consideration are well known to those of skill in the art and include: arginine and lysine; glutamate and aspartate; serine and threonine; glutamine and asparagine; and valine, leucine and isoleucine, as well as others.

Useful peptides of the invention include amino acid sequences comprising one or more of SEQ ID NO:1, 2, 4 and 6, or fragments thereof. Preferably, the peptides of the invention comprise a sequence of 30 or fewer amino acids.

The immunogenic peptides of the invention can be composite peptides having one or more of SEQ ID NO: 1, 2, 4 and 6, or fragments thereof and optionally, a non-proteasomal amino acid sequence.

Useful compositions of the invention include one or more peptides and/or fusion proteins of the invention combined with a pharmaceutically acceptable carrier and/or other pharmaceutical additive ingredients. The compositions may include an adjuvant capable of enhancing an immune response to the epitope of the peptides or fusion proteins of the invention.

The present invention may also relate to fragments of a polypeptide. Fragments, including the N-terminus of the molecule may be generated by genetic engineering of translation stop sites within a coding region. Alternatively, treatment of polypeptide with proteolytic enzymes, known as proteases, can produce a variety of N-terminal, C-terminal and internal fragments. In certain embodiments, peptides may be synthesized by known methods. Examples of fragments may include contiguous residues of 5, 6, 7, 8, 9, 10, 11, 12, 13, 14, 15, 16, 17, 18, 19, 20, 21, 22, 23, 24, 25, 30, 35, 40, 45, 50, 55, 60, 65, 75, 80, 85, 90, 95, 100, 200, 300 or more amino acids in length. These fragments may be purified according to known methods, such as precipitation (*e.g*., ammonium sulfate), HPLC, ion exchange chromatography, affinity chromatography (including immunoaffinity chromatography) or various size separations (sedimentation, gel electrophoresis, gel filtration).

### B. Synthetic Peptides

Various embodiments of the invention describe peptides or polypeptides for use in methods and compositions for regulating and/or diagnosing fertility. In some embodiments the production of anti-proteasomal or anti-ubiquitin antibodies for use in inhibiting or reducing as well as detecting fertility in an animal are contemplated. Peptides of the invention may also be synthesized in solution or on a solid support in accordance with conventional techniques. Various automatic synthesizers are commercially available and can be used in accordance with known protocols. See, for example, Stewart and Young (1984); Tam *et al.* (1983); Merrifield (1986); and Barany and Merrifield (1979). Short peptide sequences, or libraries of overlapping peptides, usually from about 6 up to about 35 to 50 amino acids, which correspond to peptides described herein, can be readily synthesized. In some embodiments, recombinant DNA technology may be employed wherein a nucleotide sequence which encodes a peptide or polypeptide of the invention is inserted into an expression vector, transformed or transfected into an appropriate host cell and cultivated under conditions suitable for expression.

### C. Fusion Peptides or Polypeptides

A specialized kind of insertional variant is the fusion protein. This molecule generally has all or a substantial portion of a first molecule, linked at the N- or C-terminus, to all or a portion of a second polypeptide. For example, fusions typically employ leader sequences from other species to permit the recombinant expression of a protein in a heterologous host. Another useful fusion includes the addition of an immunologically active domain, such as an antibody epitope, to facilitate purification of the fusion protein. Inclusion of a cleavage site at or near the fusion junction will facilitate removal of the extraneous polypeptide after purification. Other useful fusions include linking of functional domains, such as active sites from enzymes, glycosylation domains, cellular targeting signals or transmembrane regions. Other fusions of the invention include a fusion of two or more proteasomal antigens. In certain embodiments the two or more proteasomal antigens are reversibly or irreversibly coupled to each other.

### D. Purification of Peptides or Polypeptides

In certain embodiments, it may be desirable to purify a peptide or polypeptide. Protein purification techniques are well known to those of skill in the art. These techniques involve, at one level, the crude fractionation of the cellular milieu to polypeptide and non-polypeptide fractions. Having separated the polypeptide from other molecules, the polypeptide of interest may be further purified using chromatographic and electrophoretic techniques to achieve partial or complete purification (or purification to homogeneity). Analytical methods particularly suited to the preparation of a pure peptide are ion-exchange chromatography, exclusion chromatography; polyacrylamide gel electrophoresis; isoelectric focusing. A particularly efficient method of purifying peptides is fast protein liquid chromatography or even HPLC.

Various techniques suitable for use in protein purification will be well known to those of skill in the art. These include, for example, precipitation with ammonium sulphate, PEG, antibodies and the like or by heat denaturation, followed by centrifugation; chromatography steps such as ion exchange, gel filtration, reverse phase, hydroxylapatite and affinity chromatography; isoelectric focusing; gel electrophoresis; and combinations of such and other techniques. As is generally known in the art, it is believed that the order of conducting the various purification steps may be changed, or that certain steps may be omitted, and still result in a suitable method for the preparation of a substantially purified protein or peptide.

Certain aspects of the present invention concern the purification and in particular embodiments, the substantial purification, of a peptide. The term "purified peptide, polypeptide or protein" as used herein, is intended to refer to a composition, isolatable from other components, wherein the peptide is purified to any degree relative to its naturally-obtainable state. A purified protein or peptide therefore also refers to a protein or peptide, free from the environment in which it may naturally occur.

Generally, "purified" will refer to a protein or peptide composition that has been subjected to fractionation to remove various other components, and which composition substantially retains its activity. Where the term "substantially purified" is used, this designation will refer to a composition in which the protein or peptide forms the major component of the composition, such as constituting about 50%, about 60%, about 70%, about 80%, about 90%, about 95% or more of the peptides, polypeptides, or proteins in the composition. Various methods for quantifying the degree of purification of the protein or peptide will be known to those of skill in the art in light of the present disclosure.

### E. Antigens

Antigens of the invention are typically isolated or derived from one or more protein components of a mammalian proteasome, for a review see *e.g*., Schmid and Briand, 1997 or Bogyo *et al.,* 1997. In various embodiments of the invention antigens are administered to an animal to regulate the fertility of the animal or to produce antibodies for use in fertility regulation. In particular embodiments, immunization of vertebrate animals according to the present invention may include the administration of a polynucleotide encoding one or more proteasome peptides.

In some embodiments the invention relates to methods of preparing antibodies against a proteasomal antigen comprising the steps of: (a) identifying a proteasomal antigen; (b) generating an immune response in a vertebrate animal with an antigen as described herein; and (c) obtaining antibodies produced in the animal or in a hybridoma cell line prepared by the fusion of cells harvested from immunized animals with immortalized carcinoma cells.

The invention also relates to methods of preparing antibodies against a proteasomal polypeptide that is immunogenic, but not necessarily protective as a vaccine. For example proteasomal-specific antibodies might be useful in fertility evaluation, infertility diagnosis and investigations or antibody-therapy. Immunizing animals with an antigen or a polynucleotide encoding an antigen may be used to produce anti-proteasome antibodies. In other methods of producing anti-proteasomal antibodies, the identified antigen might be used for panning against a phage library. This procedure would be used to isolate antibodies *in vitro.*

### F. Antibodies

In another aspect, the present invention includes antibody compositions that are immunoreactive with components of the proteasome pathway or any portion thereof. In still other embodiments, an antigen of the invention may be used to produce antibodies and/or antibody compositions. Antibodies may be specifically or preferentially reactive to proteasome or ubiquitin polypeptides, or to polypeptides of the enzymes in ubiquitin-proteasome pathway. Exemplary antibodies include those reactive to components or associated polypeptides of the proteasome pathway includes antibodies reactive to an antigen having the sequences as set forth in SEQ ID NO:1, 2, 4 and 6, fragments, variants, or mimetics thereof, or closely related sequences. The antibodies may be polyclonal or monoclonal and produced by methods known in the art. The antibodies may also be monovalent or bivalent. An antibody may be split by a variety of biological or chemical means. Each half of the antibody can only bind one antigen and, therefore, is defined monovalent. Means for preparing and characterizing antibodies are well known in the art (see, *e.g*., Harlow and Lane, 1988).

Peptides corresponding to one or more antigenic determinants of a polypeptide of the present invention may be prepared in order to produce an antibody. Such peptides should generally be at least five or six amino acid residues in length, will preferably be about 10, 15, 20, 25 or about 30 amino acid residues in length, and may contain up to about 35 to 50 residues or so. Synthetic peptides will generally be about 35 residues long, which is the approximate upper length limit of automated peptide synthesis machines, such as those available from Applied Biosystems (Foster City, CA). Longer peptides also may be prepared, *e.g*., by recombinant means. In other methods full or substantially full length polypeptides may be used to produce antibodies of the invention.

Once a peptide(s) are prepared that contain at least one or more antigenic determinants, the peptides are then employed in the generation of antisera against the polypeptide. Minigenes or gene fusions encoding these determinants also can be constructed and inserted into expression vectors by standard methods, for example, using PCR cloning methodology. The use of peptides for antibody generation or vaccination typically requires conjugation of the peptide to an immunogenic carrier protein, such as hepatitis B surface antigen, keyhole limpet hemocyanin or bovine serum albumin. Methods for performing this conjugation are well known in the art, as are various adjuvants.

The antibodies used in the methods of the invention include derivatives that are modified, *i*.*e*, by the covalent attachment of any type of molecule to the antibody. For example, but not by way of limitation, the antibody derivatives include antibodies that have been modified, *e*.*g*., by glycosylation, acetylation, pegylation, phosphorylation, amidation, derivatization by known protecting/blocking groups, proteolytic cleavage, linkage to a cellular ligand (an other protein), etc. Any of numerous chemical modifications may be carried out by known techniques. Additionally, the derivative may contain one or more non-classical ammo acids.

For some uses, including *in vivo* use of antibodies in humans and *in vitro* detection assays, it may be preferable to use chimeric, humanized, or human antibodies. A chimeric antibody is a molecule in which different portions of the antibody are derived from different animal species or molecules, such as antibodies having a variable region derived from a murine monoclonal antibody and a constant region derived from a human immunoglobulin. Methods for producing chimeric antibodies are known in the art. See *e.g*., Morrison, 1985; Gillies *et al.* 1989; U.S. Patents 5,807,715; 4,816,567; and 4,816,397. Humanized antibodies are antibody molecules from non-human species that bind the desired antigen having one or more complementarity determining regions (CDRs) from the non-human species and framework regions from a human immunoglobulin molecule. Antibodies can be humanized using a variety of techniques known in the art including, for example, CDR-grafting (EP 239,400; WO 91/09967; U.S. Patents 5,225,539; 5,530,101 and 5,585,089), veneering or resurfacing (EP 592,106; EP 519,596; Padlan, 1991; Studnicka *et al.,* 1994; Roguska *et al.,* 1994), and chain shuffling (U.S. Patent 5,565,332).

Completely human antibodies are particularly desirable for therapeutic treatment of human patients. Human antibodies can be made by a variety of methods known in the art including phage display methods described above using antibody libraries derived from human immunoglobulin sequences. See U.S. Patents 4,444,887 and 4,710,111; and WO 98/46645; WO 99/50433; WO 98/24893; WO 98/16654; WO 96/34096; WO 96/33735; and WO 91/10741.

Human antibodies can also be produced using transgenic mice which are incapable of expressing functional endogenous immunoglobulins, but which can express human immunoglobulin genes. For an overview of this technology for producing human antibodies, see Lonberg and Huszar, 1995. For a detailed discussion of this technology for producing human antibodies and human monoclonal antibodies and protocols for producing such antibodies, see, *e*.*g*., WO 98/24893; WO 92/01047; WO 96/34096; WO 96/33735; EP 0598877; U.S. Patents 5,413,923; 5,625,126; 5,633,425; 5,569,825; 5,661,016; 5,545,806; 5,814,318; 5,885,793; 5,916,771; and 5,939,598. In addition, companies such as Abgenix, Inc. (Freemont, CA). Kirin, Inc. (Japan), Medarex (NJ) and Genpharm (San Jose, CA) can be engaged to provide human antibodies directed against a selected antigen using technology similar to that described above.

The antibodies of the invention may also be modified by the methods and coupling agents described by Davis et al. (U.S. Patent 4,179,337) in order to provide compositions that can be injected into the mammalian circulatory system with substantially no immunogenic response.

### III. POLYNUCLEOTIDES

The present invention provides compositions comprising polynucleotides and methods of using these compositions to regulate and diagnose fertility in a subject. In some embodiments the methods and compositions may be directed to the induction of an immune response in an animal or subject to proteasomes, ubiquitin or associated enzymes of a ubiquitin-proteasome pathway.

In various embodiments of the invention polynucleotides encoding proteasomal proteins, monomeric ubiquitin or ubiquitin polypeptides, as well as fragments thereof, are provided. Proteasomal proteins include, but are not limited to α1-7 and β1-7 subunits of the proteasome, in particular a MECL1 subunit. A polynucleotide encoding a proteasome polypeptide or a polypeptide fragment may be expressed in prokaryotic or eukaryotic cells. The expressed polypeptides or polypeptide fragments may be purified for use as proteasomal antigens for administration to vertebrate animals to generate antibodies immunoreactive with proteasomal polypeptides or polypeptide fragments.

The present invention is not limited in scope to a proteasomal polynucleotide or polypeptide of any particular mammal. One of ordinary skill in the art could, using the nucleic acids described herein, readily identify related homologs in other mammals. In addition, it should be clear that the present invention is not limited to the specific nucleic acids disclosed herein. As discussed below, a specific "proteasomal protein" gene or polynucleotide fragment may contain a variety of different bases and yet still produce a corresponding polypeptide that is functionally indistinguishable, and in some cases structurally indistinguishable, from the polynucleotide sequences disclosed herein. The nucleic acid sequence of a variety of proteasomal or ubiquitin sequences from various animals may be found in GenBank and other public databases.

### A. Nucleic Acids Encoding Peptides or Antigens

In some embodiments of the present invention, polynucleotides encoding antigenic proteasomal polypeptides capable of inducing an immune response in vertebrate animals and for use as an antigen to generate anti-proteasomal or anti-ubiquitin antibodies are contemplated. In certain instances, it may be desirable to express proteasomal polynucleotides encoding a particular antigenic proteasomal polypeptide domain or sequence to be used as a vaccine or in generating anti-proteasomal antibodies for passive immunization. Nucleic acids according to the present invention may encode an entire proteasome protein, or any other fragment thereof. The nucleic acid may be derived from PCR-amplified DNA of a particular organism. In other embodiments, however, the nucleic acid may comprise genomic DNA, complementary DNA (cDNA), or synthetic DNA. A protein may be derived from the designated sequences for use in a vaccine or in methods for isolating antibodies. The proteasomal polypeptide encoding genes or their corresponding cDNA may be inserted into an appropriate expression vector for the production of antigenic proteasomal polypeptides.

The term "cDNA" is intended to refer to DNA prepared using messenger RNA (mRNA) as a template. The advantage of using a cDNA, as opposed to DNA amplified or synthesized from a genomic DNA template or a non-processed or partially processed RNA template is that a cDNA primarily contains coding sequences comprising the open reading frame (ORF) of the corresponding protein. There may be times when the full or partial genomic sequence is preferred, such as where the non-coding regions are required for optimal expression.

In still further embodiments, a polynucleotide from a given animal may be represented by natural variants that have slightly different nucleic acid sequences but, nonetheless, encode the same polypeptide (see Table 1 below). In addition, it is contemplated that a given proteasomal polypeptide from an animal may be generated using alternate codons that result in a different nucleic acid sequence but encodes the same polypeptide.

As used in this application, the term "a nucleic acid encoding a proteasomal polynucleotide" refers to a nucleic acid molecule that has been isolated free of total cellular nucleic acid. The term "functionally equivalent codon" is used herein to refer to codons that encode the same amino acid, such as the six codons for arginine or serine (Table 1, below), and also refers to codons that encode biologically equivalent amino acids, as discussed in the following pages.

Allowing for the degeneracy of the genetic code, sequences are considered essentially the same as those set forth in a proteasomal gene or polynucleotide that have at least about 50%, usually at least about 60%, more usually about 70%, most usually about 80%, preferably at least about 90% and most preferably about 95% of nucleotides that are identical to the nucleotides of a given proteasomal gene or polynucleotide. Sequences that are essentially the same as those set forth in a proteasomal gene or polynucleotide may also be functionally defined as sequences that are capable of hybridizing to a nucleic acid segment containing the complement of a proteasomal polynucleotide under standard conditions. The term closely related sequences refers to sequences with either substantial sequence similarity or sequence that encode proteins that perform or invoke similar antigenic responses as described herein. The term closely related sequence is used herein to designate a sequence with a minimum or 50% similarity with a polynucleotide or polypeptide with which it is being compared.

The DNA segments of the present invention include those encoding biologically functional equivalent proteasomal proteins and peptides, as described above. Such sequences may arise as a consequence of codon redundancy and amino acid functional equivalency that are known to occur naturally within nucleic acid sequences and the proteins thus encoded. Alternatively, functionally equivalent proteins or peptides may be created via the application of recombinant DNA technology, in which changes in the protein structure may be engineered, based on considerations of the properties of the amino acids being exchanged. Changes may be engineered through the application of site-directed mutagenesis techniques or may be introduced randomly and screened later for the desired function, as described below.

Naturally, the present invention also encompasses oligonucleotides that are complementary, or essentially complementary to the sequences of a proteasomal polynucleotide. Nucleic acid sequences that are "complementary" are those that are capable of base-pairing according to the standard Watson-Crick complementary rules. As used herein, the term "complementary sequences" means nucleic acid sequences that are substantially complementary, as may be assessed by the same nucleotide comparison set forth above, or as defined as being capable of hybridizing to the nucleic acid segment of a proteasomal polynucleotide under relatively stringent conditions such as those described herein.

Alternatively, the hybridizing segments may be shorter oligonucleotides. Sequences of 17 bases long should occur only once in the human genome and, therefore, suffice to specify a unique target sequence. Although shorter oligomers are easier to make and increase *in vivo* accessibility, numerous other factors are involved in determining the specificity of hybridization. Both binding affinity and sequence specificity of an oligonucleotide to its complementary target increases with increasing length. It is contemplated that exemplary oligonucleotides of 8, 9, 10, 11, 12, 13, 14, 15, 16, 17, 18, 19, 20, 25, 30, 35, 40, 45, 50, 55, 60, 65, 70, 75, 80, 85, 90, 95, 100 or more base pairs will be used, although others are contemplated. Longer polynucleotides encoding 250, 500, 1000, 1212, 1500, 2000, 2500, 3000 or 3500 bases and longer are contemplated as well. Such oligonucleotides or polynucleotides will typically find use, for example, as probes in Southern and Northern blots and as primers in amplification reactions, or for vaccines.

**TABLE 1**

| **Amino Acids** | | | **Codons** | | | | | |
|---|---|---|---|---|---|---|---|---|
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

### C. Non-bacterially amplified nucleic acids

A nucleic acid or polynucleotide of the invention may be made by any technique known to one of ordinary skill in the art, such as for example, chemical synthesis, or enzymatic production. In the methods of the present invention, one or more oligonucleotide or polynucleotide may be used. Various different mechanisms of oligonucleotide synthesis have been disclosed in for example, U.S. Patent Nos. 4,659,774, 4,816,571, 5,141,813, 5,264,566, 4,959,463, 5,428,148, 5,554,744, 5,574,146, and 5,602,244.

A non-limiting example of an enzymatically produced nucleic acid or polynucleotide includes one produced by enzymes in amplification reactions such as PCR^{™} (see for example, U.S. Patent No. 4,683,202 and U.S. Patent No. 4,682,195), or the synthesis of an oligonucleotide described in U.S. Patent No. 5,645,897.

Another method for nucleic acid or polynucleotide amplification is the ligase chain reaction ("LCR"), disclosed in EPO No. 320 308. U.S. Patent 4,883,750 describes a method similar to LCR for binding probe pairs to a target sequence. Methods based on ligation of two (or more) oligonucleotides in the presence of nucleic acid having the sequence of the resulting "di-oligonucleotide", thereby amplifying the di-oligonucleotide, may also be used in the amplification step of the present invention. Wu *et al.,* (1989).

### D. Polynucleotide Delivery

In certain embodiments of the invention, an expression construct comprising a proteasomal polynucleotide or polynucleotide segment under the control of a promoter operable in eukaryotic cells is provided. The general approach in certain aspects of the present invention is to provide a cell with an expression construct encoding a specific protein, polypeptide or peptide fragment, thereby permitting the expression of the antigenic protein, polypeptide or peptide fragment in the cell. Following delivery of an expression construct, the protein, polypeptide or peptide fragment encoded by the expression construct is synthesized by the transcriptional and translational machinery of a cell. Various compositions and methods for polynucleotide delivery are known (see Sambrook *et al.,* 2001; Liu and Huang, 2002; Ravid *et al.,* 1998; Balicki and Beutler, 2002.

Viral and non-viral delivery systems are two of the various delivery systems for the delivery of an expression construct encoding an antigenic protein, polypeptide, polypeptide fragment. Both types of delivery systems are well known in the art and are briefly described below. There also are two primary approaches utilized in the delivery of an expression construct for the purposes of genetic immunization; either indirect, *ex vivo* methods or direct, *in vivo* methods. *Ex vivo* gene transfer comprises vector modification of (host) cells in culture and the administration or transplantation of the vector modified cells to a subject. *In vivo* gene transfer comprises direct introduction of the vaccine vector into the subject to be immunized.

In various embodiments, a nucleic acid to be expressed may be in the context of a linear expression elements ("LEEs") and/or circular expression elements ("CEEs"), which typically encompass a complete set of gene expression components (promoter, coding sequence, and terminator). These LEEs and CEEs can be directly introduced into and expressed in cells or an intact organism to yield expression levels comparable to those from a standard supercoiled, replicative plasmid (Sykes and Johnston, 1999).

### 1. Non-Viral Polynucleotide Delivery

In one embodiment of the invention, a polynucleotide expression construct may include recombinantly-produced DNA plasmids or *in* vitro-generated DNA. In various embodiments of the invention, an expression construct comprising, for example, a proteasomal or ubiquitin polynucleotide is administered to an organism or subject via injection and/or particle bombardment (*e.g*., a gene gun)(Klein *et al.,* 1987 and Sanford *et al.,* 1991). In some embodiments administration a polynucleotide may be via intramuscular, intravenous, subcutaneous, intradermal, or intraperitoneal injection.

Transfer of an expression construct comprising proteasomal or similar polynucleotides of the present invention also may be performed by any of the methods which physically or chemically permeabilize the cell membrane (*e*.*g*., calcium phosphate precipitation, DEAE-dextran, electroporation, direct microinjection, DNA-loaded liposomes and lipofectamine-DNA complexes, cell sonication, gene bombardment using high velocity microprojectiles and receptor-mediated transfection. In certain embodiments, the use of lipid formulations and/or nano-capsules is contemplated for the introduction of a proteasomal polynucleotide, proteasomal polypeptide, or an expression vector comprising a proteasomal polynucleotide into host cells (see exemplary methods and compositions in Bangham *et al.* (1965), Gregoriadis (1979), Deamer and Uster (1983), Szoka and Papahadjopoulos (1978), Nicolau *et al.,* 1987 and Watt *et al.,* 1986; each of which is ated herein by reference). In another embodiment of the invention, the expression construct may simply consist of naked recombinant DNA, expression cassettes or plasmids.

### 2. Viral Vectors

In certain embodiments, it is contemplated that a polynucleotide or the encoded polypeptide that confers an immune response pursuant to the invention may be delivered by a viral vector. In particular embodiments, the immune response may be to a proteasome or ubiquitin peptide or polypeptide. The capacity of certain viral vectors to efficiently infect or enter cells, to integrate into a host cell genome and stably express viral genes, have led to the development and application of a number of different viral vector systems (Robbins *et al.,* 1998). Viral systems are currently being developed for use as vectors for *ex vivo* and *in vivo* gene transfer. For example, adenovirus, herpes-simplex virus, retrovirus and adeno-associated virus vectors are being evaluated currently for treatment of diseases such as cancer, cystic fibrosis, Gaucher disease, renal disease and arthritis (Robbins and Ghivizzani, 1998; Imai *et al.,* 1998; U. S. Patent 5,670,488).

In particular embodiments, an adenoviral (U.S. Patents. 6,383,795, 6,328,958 and 6,287,571), retroviral (U.S. Patents. 5,955,331; 5,888,502, 5,830,725), Herpes-Simplex Viral (U.S. Patents. 5,879,934; 5,851,826), Adeno-associated virus (AAV), poxvirus; *e.g*., vaccinia virus (Gnant *et al.,* 1999a; Gnant *et al.,* 1999b), alpha virus; *e.g*., sindbis virus, Semliki forest virus (Lundstrom, 1999), reovirus (Coffey *et al.,* 1998) and influenza A virus (Neumann *et al.,* 1999), Chimeric poxviral/retroviral vectors (Holzer *et al.,* 1999), adenoviral/retroviral vectors (Feng *et al.,* 1997; Bilbao *et al.,* 1997; Caplen *et al.,* 1999) and adenoviral/adeno-associated viral vectors (Fisher *et al.,* 1996; U. S. Patent 5,871,982), expression vectors are contemplated for the delivery of expression constructs. "Viral expression vector" is meant to include those constructs containing virus sequences sufficient to (a) support packaging of the construct and (b) to ultimately express a tissue or cell-specific construct that has been cloned therein. Virus growth and manipulation is known to those skilled in the art.

### E. Expression vectors

Polynucleotide encoding a proteasomal peptide may be cloned in a genetic immunization vector or any other suitable expression construct. The vector may comprise a promoter operable in eukaryotic cells, for example a CMV promoter, or any other suitable promoter. In such methods, the polynucleotide may be administered by an intramuscular injection, intradermal injection, or epidermal injection or particle bombardment. The polynucleotide may likewise be administered by intravenous, subcutaneous, intralesional, intraperitoneal, oral, other mucosal or inhaled routes of administration. In some specific, exemplary embodiments, the administration may be via epidermal injection/bombardment of at least 0.005 µg to 5.0 µg of the polynucleotide. In some cases, a second administration, for example, an intramuscular injection and/or epidermal injection may be administered at least about two weeks or longer after the first administration. In these methods, the polynucleotide may be, but need not be, cloned into a viral expression vector, for example, a viral expression vector, including adenovirus, herpes-simplex virus, retrovirus or adeno-associated virus vectors. The polynucleotide may also be administered in any other method disclosed herein or known to those of skill in the art. One or more polynucleotides can be comprised in one or more expression vectors.

Vectors suitable for expression are known, and include, but are not limited to, bacterial expression vectors, yeast expression vectors, and baculovirus vectors. The proteasomal peptides are expressed in a host cell, when the host cell is cultured under suitable conditions. Host cells can include, but are not limited to, silk worm larvae, CHO cells, *E. coli,* and yeast. Methods of recovery of peptides expressed in host cells are known. Vectors, hosts, methods of expressing vectors and recovering peptides are known (see, for example, Sambrook *et al.,* 2001 and O'Reilley *et al.,* 1994.

### IV. IMMUNIZATION

An immune response may be elicited to various peptides or polypeptides described herein, including by not limited to proteasome and ubiquitin peptides or polypeptides. The immunogenic peptides derived from proteasome or ubiquitin peptides of the invention are preferably used to induce anti-proteasome or anti-ubiquitin antibodies, respectively. For example, a fusion protein comprising one or more proteasomal epitope, with or without a non-proteasomal immunogen, is administered to an animal, for example by injection. The immunogen may be delivered with an adjuvant, as known in the field. The course of administration may be one or multiple boosters, as needed to induce anti-proteasomal antibody titer. Thereafter, maintenance doses can be administered as required to retain a sufficient antibody titer. The appropriate dose and immunization regimen for the peptide, peptide composition, or vaccine of the invention can be determined by one of skill in the art, armed with the information provided in this text and the Examples below.

The concept of vaccination/immunization is based on two fundamental characteristics of the immune system, namely specificity and memory of immune system components. Vaccination/immunization will initiate a response specifically directed to the antigen with which a subject was challenged. Furthermore, a population of memory B and T lymphocytes may be induced. Upon re-exposure to the antigen(s) the immune system will be primed to respond much faster and much more vigorously, thus endowing the vaccinated/immunized subject with immunological protection against an antigen. An immune response may be augmented by administration of the same or different antigen repeatedly to a subject or by boosting a subject with a vaccine composition.

Vaccination is the artificial induction of actively-acquired immunity by administration of all or part of an antigen. In addition to actively-acquired immunity, passive immunization methods may also be used to provide a therapeutic benefit to a subject, see below.

In particular, genetic vaccination, also known as DNA immunization, involves administering an antigen-encoding expression vector(s) in vivo, in vitro, or ex vivo to induce the production of a correctly folded antigen(s) within an appropriate organism, tissue, cell or a target cell(s). The expressed proteins or peptides will typically be displayed on the cellular surface of the transfected cells in conjunction with the Major Histocompatibility Complex (MHC) antigens of the normal cell. The display of these antigenic determinants in association with the MHC antigens is intended to elicit the proliferation of cytotoxic T-lymphocyte clones specific to the determinants. Furthermore, the proteins released by the expressing transfected cells can also be picked up, internalized, or expressed by antigen-presenting cells to trigger a systemic, humoral antibody response.

A vaccine is a composition including an antigen derived from all or part of an immunogenic agent, or a mimetic thereof that is modified to make it non-pathogenic and suitable for use in vaccination. Types of vaccines include, but are not limited to genetic vaccines, virosomes, attenuated or inactivated whole organism vaccines, recombinant protein vaccines, conjugate vaccines, transgenic plant vaccines, toxoid vaccines, purified sub-unit vaccines, multiple genetically-engineered vaccines, anti-idiotype vaccines and other vaccine types known in the art.

An immune response may be an active or a passive immune response. Active immunity develops when the body is exposed to various antigens. It typically involves B or T lymphocytes. B lymphocytes (also called B cells) produce antibodies. Antibodies attach to a specific antigen and make it easier for phagocytes to destroy the antigen. Typically, T lymphocytes (T cells) attack antigens directly and may provide some control over the immune response. B cells and T cells develop that are specific for a particular antigen or antigen type. Passive immunization generally refers to the administration of preformed antibodies or other binding agents, which bind an antigen(s).

In certain cases, an immune response may be a result of adoptive immunotherapy. In adoptive immunotherapy lymphocyte(s) are obtained from a subject and are exposed or pulsed with an antigenic composition. For exemplary methods or compositions see U.S. Patents 5,614,610; 5,766,588; 5,776,451; 5,814,295; 6,004,807 and 6,210,963).

The present invention includes methods of immunizing, treating or vaccinating a subject by contacting the subject with an antigenic composition comprising a proteasomal antigen or a polynucleotide encoding a proteasomal antigen, a ubiquitin antigen, or an antigen derived from an enzyme active in a ubiquitin-proteasome pathway. An antigenic composition may comprise a nucleic acid; a polypeptide; an attenuated pathogen, such as a virus, a bacterium, a fungus, or a parasite, which may or may not express a proteasomal antigen; a prokaryotic cell expressing a proteasomal antigen; a eukaryotic cell expressing a proteasomal antigen; a virosome; and the like, or a combination thereof. As used herein, an "antigenic composition" will typically comprise an antigen in a pharmaceutically acceptable formulation.

Antigen refers to any substance, molecule, or molecule encoding a substance that an organism elicits an immune response, particularly in the form of specific antibodies or cell types reactive to an antigen. An antigenic composition may further comprise an adjuvant, an immunomodulator, a vaccine vehicle, and/or other excipients, as described herein and is known in the art (for example, see Remington's Pharmaceutical Sciences).

Various methods of introducing an antigen or an antigen composition to a subject are known in the art. Vaccination methods include, but are not limited to DNA vaccination or genetic immunization (for examples see U.S. Patents 5,589,466, 5,593,972, 6,248,565, 6,339,086, 6,348,449, 6,348,450, 6,359,054, each of which is ated herein by reference), edible transgenic plant vaccines (for examples see U.S. Patents 5,484,719, 5,612,487, 5,914,123, 6,034,298, 6,136,320, and 6,194,560, each of which is ated herein by reference), transcutaneous immunization (Glenn *et al.,* 1999 and U.S. Patent 5,980,898, each of which is ated herein by reference), nasal or mucosal immunization (for examples see U.S. Patents 4,512,972, 5,429,599, 5,707,644, 5,942,242, each of which is ated herein by reference); virosomes (Huang *et al.,* 1979; Hosaka *et al.,* 1983; Kaneda, 2000; U.S. Patents 4,148,876; 4,406,885; 4826,687; 5,565,203; 5,910,306; 5,985,318, each of which is ated herein by reference) , live vector and the like. Antigen delivery methods may also be combined with one or more vaccination regimes.

Vaccines comprising an antigen, a polypeptide or a polynucleotide encoding an antigen may present an antigen in a variety of contexts for the stimulation of an immune response. Some of the various vaccine contexts include attenuated pathogens, inactivated pathogens, toxoids, conjugates, recombinant vectors, and the like. Polypeptides of the invention may be mixed with, expressed by or coupled to various vaccine compositions. Various vaccine compositions may provide an antigen directly or deliver an antigen producing composition, *e.g*., an expression construct, to a cell that subsequently produces or expresses an antigen or antigen encoding molecule.

Vaccines of the invention comprise an effective therapeutic dose of an immunogenic proteasomal or ubiquitin peptides sufficient to induce the production of anti-proteasomal or anti-ubiquitin antibodies. The resulting immunization may result in a contraceptive effect. Preferably, the vaccine is effective to produce a contraceptive effect in one or more mammals. Most preferably, the vaccine produces anti-proteasome antibodies when administered to rodents or non-rodent mammals, including one or more of rabbits, pigs, horses, monkeys, dogs, cats, cows, and humans.

In other embodiments, proteinaceous compositions or polypeptides, fragments or mimetics thereof, may be used to produce anti-idiotypic antibodies for use in a vaccine. In an anti-idiotype vaccine the immunogen is an antibody against the Fab end of a second antibody which was raised against an antigenic molecule of a pathogen. The Fab end of the first antibody will have the same antigenic shape as the antigenic molecule of the pathogen and may then be used as an antigen (see exemplary U.S. Patents 5,614,610, 5,766,588). "Humanized" antibodies for use herein may be antibodies from non-human species wherein one or more selected amino acids have been exchanged for amino acids more commonly observed in human antibodies. This can be readily achieved through the use of routine recombinant technology, particularly site-specific mutagenesis. Humanized antibodies may also be used as a passive immunization agent as described below.

### V. PHARMACEUTICAL COMPOSITIONS

Pharmaceutical aqueous compositions of the present invention comprise an effective amount of one or more proteasome inhibitors dissolved or dispersed in a pharmaceutically acceptable carrier or aqueous medium. The phrases "pharmaceutically or pharmacologically acceptable" refer to molecular entities and compositions that do not produce an adverse, allergic or other untoward reaction when administered to a human, unless specifically designed to elicit such a response. As used herein, "pharmaceutically acceptable carrier" includes any and all solvents, dispersion media, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be ated into the compositions.

The actual dosage amount of a composition of the present invention administered to a subject can be determined by physical and physiological factors such as body weight and on the route of administration. With these considerations in mind, the dosage of a peptide, polypeptide, polynucleotide, immunoglobulin or proteasome inhibitor composition for a particular subject and/or course of treatment can readily be determined.

Proteosome inhibitors that may be used with the invention are known in the art and available commercially, for example, from BIOMOL Research Laboratories, Inc., Plymouth Meeting, PA. Examples of such inhibitors that may find use with the invention include N-Acetyl-Leu-Leu-Nle-H, N-Acetyl-Leu-Leu-Met-H, Aclacinomycin A (Aclarubicin), Ada-(Ahx)3-(Leu)3-vinyl sulphone, Ada-Lys(biotinyl)-(Ahx)3-(Leu)3-vinyl sulphone, Ada-Tyr-(Ahx)3-(Leu)3-vinyl sulphone, Bactenecin-5, Brefeldin A, Curcumin, (-)-Epigallocatechin gallate (EGCG), Epoxomicin, Gliotoxin, Lactacystin, clasto-Lactacystin â-lactone, N-Tosyl-Lys-chloromethylketone (TLCK), N-Tosyl-Phe-chloromethylketone (TPCK), NIP-(Leu)3-vinyl sulphone, Phepropeptin A chymotrypsin, Phepropeptin B chymotrypsin, Phepropeptin C chymotrypsin, Phepropeptin D chymotrypsin, PR11, PR39, Ubiquitin5+1, Z-Ile-Glu(OBut)-Ala-Leu-H (PSI), Z-Leu-Leu-Leu-vinyl sulphone, Z-Leu-Leu-Nva-H (MG115), Z-Leu-Leu-Leu-H (MG132), Z-Leu-Leu-Leu-B(OH)2 (MG262), and Z-Leu-Leu-Tyr-COCHO.

Pharmaceutical compositions of the present invention can be administered intravenously, intradermally, intraarterially, intraperitoneally, intraarticularly, intrapleurally, intratracheally, intranasally, intravaginally, topically, intramuscularly, intraperitoneally, subcutaneously, intravesicularlly, mucosally, orally, topically, locally using aerosol, injection, infusion, continuous infusion, localized perfusion bathing target cells directly or via a catheter or lavage. Typically, such compositions are prepared as injectables, either as liquid solutions or suspensions; solid forms suitable for preparing solutions or suspensions upon the addition of a liquid prior to injection can also be prepared; and the preparations can also be emulsified. The compositions will be sterile, a fluid to the extent that easy syringability exists, stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein.

Although it is most preferred that compositions of the present invention be prepared in sterile water containing other non-active ingredients, made suitable for injection, solutions of such active ingredients can also be prepared in water suitably mixed with a surfactant, such as hydroxypropylcellulose, if desired. Dispersions can also be prepared in liquid polyethylene glycol and mixtures thereof, and in oils. The carrier can also be a solvent or dispersion medium containing, for example, water, ethanol, polyol (for example, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants.

The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Upon formulation, solutions will be administered in a manner compatible with the dosage formulation and in such amount as is effective for inducing contraceptive effect. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

### VI. EXAMPLES

### EXAMPLE 1

### MATERIALS AND METHODS

### A. Antibodies

Proteasome subunit sampler pack and the following rabbit sera against proteasomal subunits were purchased from Affinity Research products. Ltd., Mammhead, UK: Anti-MECL1 subunit serum (cat. #PW8150), raised against recombinant peptide based on the mouse MECL-1-amino acid sequence of AA 218-236 (NVDACVITAG GAKLQRALSTPTEPVQRAGR (SEQ ID NO:1); Hayashi *et al.,* 1997), used at 1/200 for immunofluorescence and immunogold transmission electron microscopy (TEM), 1/4000 for western blotting and 1/2000 to 1/8000 for inhibiting in vitro fertilization. This antibody was shown by the manufacturer to recognize the 29 kDa MECL-1 subunit as well as several other, larger protein complexes containing this subunit. Anti-α/β-subunit serum (cat. # PW 8155) was raised against proteasomal preparation isolated from human red blood cells (Tanaka and Tsurumi, 1997), recognizing a set of bands between 25-30 kDa in human erythrocyte lysate, and additional, higher MW-bands in other cell lysates, corresponding to several distinct α-type and β-type subunits. Anti-β1i-subunit serum (cat. #PW 8205) was raised against recombinant, full length sequence of murine β1i subunit (Groettrup *et al.,* 1996) and recognizes proteins of 26kDa, 36 kDa and possibly other higher MW protein complexes. The α/β and β1i anti-sera were used at the dilution of 1/200 for immunofluorescence and immunogold TEM, and at 1/4000 for western blotting.

Ubiquitin on the outer face of porcine zona pellucida was detected by antibody KM691 (dilution of 1/200 for immunofluorescence, 1:1000 for western; purchased from Kamiya Biomedical Company, Seattle, WA), raised against the whole molecule of recombinant human ubiquitin. Pronuclei were counterstained using rabbit polyclonal antibody AB 1690 (dilution of 1/100), recognizing porcine embryonic-nuclear ubiquitin-CEP 52 tail fusion protein (McCauley et al, 2002), purchased from Chemicon International Inc., Temecula, CA). This antibody also recognized ubiquitinated proteins in the outer layer of ZP on the ovarian tissue sections (FIG. 3 F).

The acrosomal tyrosine kinase c-Yes, a marker of acrosomal exocytosis (Leclerc and Goupil, 2002), was detected using rabbit serum against a recombinant peptide corresponding to the evolutionarily conserved C-terminal sequence of human c-Yes (dil., 1/100), purchased from Santa Cruz Biotechnology, Inc., Santa Cruz, CA. Rabbit anti prohibitin antibody Ab-2 (dil., 1/100; Thompson *et al.,* 1997), was purchased from Neomarkers Inc., Union City, CA.

Secondary antibodies used for immunofluorescence studies included the red fluorescent, TRITC-conjugated and the green-fluorescent, FITC-conjugated goat anti mouse IgG, goat anti-mouse IgM and goat anti-rabbit IgG from Zymed Inc., South San Francisco, CA, diluted to 1/80. DNA was counterstained with blue-fluorescent stain DAPI (Molecular Probes Inc., Eugene, OR). For colloidal gold TEM, 10 nm gold-conjugated goat anti-rabbit IgG was purchased from Electron Microscopy Sciences, Washington, PA, and used at a dilution of 1/10.

### B. Gametes and In Vitro Fertilization

Ovaries from pre-pubertal gilts were collected at a local slaughterhouse and transported to the laboratory for the isolation of oocytes from the 3-6 mm large antral follicles. Cumulus cell-oocyte-complexes (COCs) were selected under a dissecting microscope for the presence of multi-layered cumulus oophorus and washed repeatedly in HEPES-buffered Tyrode's lactate (TL-HEPES) medium containing 0.1% (w/v) polyvinyl alcohol (PVA). Groups of 50 COCs were matured for 22 h at 39°C and 5% CO₂ in drops of serum-free modified tissue culture medium (TCM 199; Gibco, Grand Island, NY) supplemented with 3.05 mM glucose, 0.91 mM sodium pyruvate, 0.57 mM cysteine, 10 ng/ml epidermal growth factor, 0.5 µg/ml FSH, 0.5 µg/ml LH, 0.1 % (w/v) PVA, 75 µg/ml penicillin-G and 50 µg/ml streptomycin sulfate (Abeydeera and Day, 1997). After this time, COCs were washed in modified TCM 199 without FSH or LH and matured for an additional 20 hours.

Before fertilization, cumulus cells were removed by vortexing COCs in TL-HEPES medium containing 0.1% (w/v) hyaluronidase and washed in 50 µl drops of modified Tris-buffered medium (mTBM) consisting of 113.1 mM NaCl, 3 mM KCl, 7.5 mM CaCl₂, 20 mM Tris, 11 mM glucose, 5 mM sodium pyruvate, 2 mM caffeine and 0.2% (w/v) BSA. Fertilization was performed in the 50 µl drops of mTBM medium. Cryopreserved semen was thawed in 10 ml of Dulbecco's PBS (Gibco, Grand Island, NY) supplemented with 0.1% (w/v) BSA. To determine fertilization by the presence of sperm tails and pronuclei inside the fertilized eggs, spermatozoa were pre-labeled with vital mitochondrial probe MitoTracker CMTM Ros as described previously (Sutovsky *et al.,* 2003), and the resultant zygotes were counterstained with DNA stain DAPI as described below. Spermatozoa were washed 2 times by centrifugation and added to the fertilization drops to a final concentration of 5 x 10⁵ spermatozoa/ml. Six hours after IVF, the presumptive zygotes were washed three times in a serum free TALP Hepes and processed for immunofluorescence, immunogold TEM or western blotting. To obtain zona free oocytes for western blotting, zygotes were treated for 30 sec. with protein-free TALP-HEPES containing 0.5% w/v Pronase (Protease, Sigma).

### C. Proteasomal Inhibitor and Antibody Treatments During IVF

To examine the role of the ubiquitin-proteasome pathway in sperm-zona interactions, the effect of anti-proteasomal antibody MECL-1, and specific inhibitors of proteasomal proteolytic activity, MG132 (Z-Leu-Leu-Leu-CHO; reversible inhibitor; Biomol Research Labs Inc, Plymouth Meeting, PA ) and lactacystin (C₁₅H₂₄N₂O₇S; irreversible inhibitor; also from Biomol), was evaluated. IVF was performed as described above with the addition of MG132 (10 µM) or lactacystin (10 µM or 100 µM) or antibody MECL-1 (dil. 1/2000, 1/4000, 1/8000; Affinity Research, Mamhead, UK) in the fertilization medium. Controls without inhibitor included addition of the appropriate solvent (MG132: 100% ethanol; lactacystin: H₂O) at equivalent dilutions to the inhibitors during IVF. Controls for antibody block included antibody omission and addition of an equivalent amount of pre-immune rabbit serum.

Because the inclusion of MG132, lactacystin or MECL-1 antibody completely prevented fertilization of zona-enclosed oocytes, additional experiments were performed to identify the exact step of fertilization at which the block occurred. IVF was performed using zona-enclosed and zona-free (ZF or ZP-) oocytes in the presence or absence of MG132 and antibody MECL-1. Removal of the zona pellucida was accomplished by a brief (30 sec) exposure of oocytes to 0.5% pronase (Sigma, St. Louis, MO). Oocytes were washed extensively and ZF oocytes were allowed to recover from enzymatic treatment for 1 h prior to insemination. For IVF, ZF oocytes were co-incubated with 1x10⁵ sperm/ml while zona-enclosed/zona-intact (ZI or ZP+) oocytes were treated as described above. Oocytes were fixed 12 h post insemination (p.i.) in 25% acetic acid/ethanol and stained with 1% orcein to determine the incidence of fertilization. In some experiments, oocytes were evaluated by immunofluorescence microscopy, detecting the presence of pronuclei and sperm tails in the zygotic cytoplasm. Zona-enclosed and zona free embryos were also fixed at 6 or 20 h p.i. and processed for immunofluorescence analysis to determine if the inhibition of proteasome activity by MG132 specifically blocked sperm-zona interaction, inhibited sperm-oolemma fusion, or affected sperm motility, viability or the ability of spermatozoa to undergo the acrosomal exocytosis.

To evaluate sperm-zona binding and sperm acrosomal status, zona-enclosed oocytes were fixed and the tyrosine kinase c-Yes, known to be present in the subacrosomal and outer acrosomal layer of perinuclear theca in spermatozoa with an intact acrosome (Leclerc and Goupil, 2002) was evaluated by immunofluorescence. The number of spermatozoa bound to the zona pellucida was determined after staining with DAPI. Five eggs per group were counterstained with an antibody against the acrosomal tyrosine kinase c-Yes (see *Immunofluorescence*) and DAPI and photographed at 60x magnification. ZP-bound acrosome-reacted and acrosome intact spermatozoa were counted and the values were expressed as a percent (%) of acrosome intact sperm/egg. Treatment results were compared by χ2 test and by general linear model procedures of SAS 8.2.

### D. Immunofluorescence on Ovarian Tissue-Sections, Isolated Spermatozoa and Whole-Mounted Zygotes.

Pieces of ovarian tissue were fixed in 4% paraformaldehyde, washed and embedded in paraffin following conventional histochemical protocols. Four micron thick paraffin tissue sections were cut, placed on microscopy slides, dewaxed by xylene, rehydrated in a 100-70% ethanol series and water, pretreated for 20 min in citric acid buffer (pH 6.0) using a steamer and blocked and processed with antibodies on a histological tray filled with a water-soaked tissue. Spermatozoa were pelleted from semen samples by centrifugation, attached to poly-L-lysine coated coverslips, fixed for 40 min. in 2% formaldehyde and processed by a passage from well to well in four well Petri dishes. Zona free and zona intact oocytes were fixed in a solution of 2% formaldehyde in PBS without attachment to slides or coverslips, handled using Unapette pipettes (Beckton Dickinson) attached to a 1 cc syringe, and processed by a passage through individual wells of a nine well Pyrex glass plate (Fischer Scientific). All samples were processed with the antibodies described above and in the *Results* section.

In a general protocol, spermatozoa on coverslips and unattached oocytes were permeabilized for 40 min or overnight in 0.1% Triton X-100, while the slides with paraffin sections were not permeabilized. All samples were blocked in 5% normal goat serum (NGS; Sigma) in PBS with 0.1% TX-100. Subsequent incubations and washes were performed using a labeling solution of neutral PBS enriched with 1% NGS and 0.1% TX-100. Coverslips were overlaid for 40 min (whole cell mounted on coverslips) or 2 h (tissue sections mounted on slides) with primary antibodies, listed above and in the *Results* section, diluted in labeling solution. After a brief wash in labeling solution, appropriate fluorescently conjugated anti-mouse IgG or anti-mouse IgM were mixed with a differentially fluorescent anti-rabbit IgG and with 2.5 µg/ml DAPI (Molecular probes Inc., Eugene, OR) and incubated with the samples for 30-40 minutes. Following a wash in labeling solution, coverslips were mounted on microscopy slides, sealed with a transparent nail polish and viewed in the Nikon Eclipse 800 epifluorescence microscope using appropriate filter sets and DIC optics. Images were acquired with a CoolSnap CCD HQ camera (Roper Scientific, Tucson, AZ) and MetaMorph software (Universal Imaging Corp., Downington, PA), archived on recordable CDs, edited using Adobe Photoshop 5.5 (Adobe Systems, Mountain View, CA) and printed on an Epson Stylus 1280 photo printer.

**E. Colloidal Gold Immuno-Labeling and Transmission Electron Microscopy.**

Zona-intact zygotes were fixed at 6 h p.i. in 4% formaldehyde as described for immunofluorescence, washed, permeabilized, blocked and incubated for 2 h with rabbit sera against proteasomal subunits or with serum against c-Yes tyrosine kinase. After a wash, zygotes were incubated for 1 h with goat anti-rabbit IgG (dil. 1/10 in PBS with 1% NGS and 0.1% TX-100), washed again and re-fixed for electron microscopy embedding in paraformaldehyde and glutaraldehyde (see below). Zygotes for ultrastructural studies were fixed for TEM without immunofluorescence fixation and processing.

For electron microscopic embedding, labeled and unlabelled zygotes were fixed in a mixture of 2% paraformaldehyde and 0.6% glutaraldehyde in cacodylate buffer, post-fixed in 1% osmium tetroxide, dehydrated by an ascending ethanol series (30-100%) and embedded in PolyBed 812 resin. Ultrathin sections were prepared on a Leica Ultracut UCT ultramicrotome, placed on 100 MESH copper grids and stained in two steps with uranyl acetate and lead citrate. Serial sections were examined and photographed in a Jeol 1200 EX electron microscope. Ten oocytes were processed for each antibody and for the ultrastructural studies of unprocessed/unlabeled zygotes. Negatives were scanned by an Umax Magic Scan flat bed scanner, recorded on a CD-R and printed on a Epson Stylus 1280 photo printer using Adobe Photoshop 5.5 software.

### F. SDS-PAGE and Western Blotting.

SDS-PAGE and Western blotting were carried out as generally described in Sambrook *et al.* (2001). Oocytes and zygotes were removed from culture at given time points, washed repeatedly in a protein-free TALP HEPES and 350 zygotes per group were boiled for 5 min in 10 µl of loading buffer containing Tris (50 mM), β-mercaptoethanol (5%), glycerol (10%), SDS (2%) and PMSF (100 mM). Boar sperm proteins were extracted by grinding in extraction buffer (50 mM Tris, 20 mM imidazole, 1 mM EDTA, 5 mM benzamidine HCl, 5 µg/ml leupeptine, 1µg/ml pepstatin A, 100 mM PMSF and 0.1% SDS), centrifuged at 17000 X g for 30 min. β - mercaptoethanol, glycerol, SDS were added to the supernatants, achieving the final concentrations as that of the loading buffer. Human spermatozoa were boiled with loading buffer, followed by centrifugation at 25000 X g for 30 min to remove the DNA pellet. Electrophoretic separation was achieved on a 10% gel, and proteins were transferred to PVDF membranes using Mini-Tank Electroblotter (Owl Scientific, Woburn, MA). The membranes were blocked with 1% caseine solution. After incubating overnight with the primary anti-ubiquitin antibodies and then with the appropriate biotin-conjugated secondary antibodies, the signals were detected with alkaline phosphatase reaction, using Vectastain ABC-AmP kit (Vector Lab Inc., Burlingame, CA).

### EXAMPLE 2

### PROTEASOMAL INHIBITORS BLOCK ZONA PENETRATION BY THE BOAR SPERMATOZOA WITHOUT AFFECTING SPERM-ZONA BINDING AND ACROSOME REACTION

It was first observed that proteasomal inhibitors lactacystin and MG132 prevent fertilization in the course of studies aimed at confirming the proteasomal route of degradation of the paternal, sperm mitochondria inside the porcine zygote (Sutovsky *et al.,* 2003). This study was followed up on by experiments designed to determine at which step of fertilization the inhibitors exert the block. Both MG132 and lactacystin prevented fertilization when added at the time of insemination. The experiment was repeated four times with consistent results. In these trials, MG132 prevented fertilization in 100% of oocytes. Lactacystin known to be less potent than MG132 and irreversible (Goldberg *et al.,* 1995) had a dose-dependent effect on fertilization rates, reaching a 100% block at 100 µm concentration. Further studies were performed with MG132 only, due to its known proteasome-specificity and reversibility (Lee and Goldberg, 1998), and the concern that lactacystin might partially inhibit the activities of some of the non-proteasomal proteases that could be present in the acrosome, namely that of cathepsin A (Ostrowska *et al.,* 2000). To rule out that MG132 affects sperm viability, sperm-zona binding or acrosomal exocytosis, oocytes fertilized in the presence of 10 µM MG132 were fixed with their zona pellucida intact at 20 h p.i. Sperm heads bound to ZP were detected with DAPI and intact acrosomes were detected by antibodies against tyrosine kinase c-Yes, known to reside in the subacrosomal and outer acrosomal layer of perinuclear theca of acrosome-intact spermatozoa (FIG. 1 A-D). Since the spermatozoa in this experiment were pre-labeled with MitoTracker CMTM Ros, the sperm mitochondrial sheaths were rendered detectable inside the fertilized oocytes. Similar to previous experiments with MG132, the inhibitor prevented fertilization completely (0% fertilization in MG132 group vs. 77% fertilization in control). However, there was no significant difference in the number of sperm bound per egg (99.3 with MG132 vs. 93.3 in control) or in the percentage of acrosome-intact, zona-bound sperm (2.01% with MG132 vs. 2.14% in control).

### EXAMPLE 3

### PROTEASOMAL SUBUNITS ARE PRESENT IN THE BOAR SPERM ACROSOME.

Taking into account the inhibitor data and previous reports confirming that proteasomal subunits are present in the mammalian/human sperm acrosome (*e.g*., Bialy *et al.,* 2001), as well as the evidence implicating the ubiquitin-proteasome system in the acrosomal activity of invertebrate animals (Matsumura and Aketa, 1991; Sawada *et al.,* 2001a, 2001b), it was decided to investigate the possibility that proteasomes could be present in the boar sperm acrosome. Polyclonal antibodies against the core subunits and subunits LMP-2 (β1i), MECL 1 and MECL 2 indeed rendered a predominant signal in the acrosome with little or no background in other sperm structures (FIG. 1 E-I). Ubiquitin was also detected in the acrosome (FIG. 1 J). The same proteasomal subunits were further detected by Western blotting in the extracts of boar sperm heads and whole boar spermatozoa (FIG. 2 A-C). Consistent with this new role of proteasomes in zona penetration, low doses of antibody against the recombinant peptide from AA sequence of murine proteasomal subunit MECL-1 (AA 218-236) reliably blocked fertilization in zona-intact, but not in the zona-free oocytes.

### EXAMPLE 4

### UBIQUITINATED PROTEINS ARE PRESENT ON THE OUTER FACE OF PORCINE ZONA PELLUCIDA.

If proteasomal inhibitors block zona penetration, and both proteasomal subunits and components of ubiquitin pathway are present in the acrosome (FIG. 1 E-J), either the ubiquitination-susceptible proteins/sperm receptors should be present on the outer layer of ZP, as shown in the *Ascidian* viteline envelope (Sawada *et al.,* 2002a), or there should be proteins/sperm receptors on the ZP, that are already ubiquitinated prior to the contact with the sperm acrosomal exudates. Using antibody KM 691 against recombinant human ubiquitin, paraffin sections of porcine ovarian tissue were screened (FIG. 3 A-D, F, G) as well as whole mounts of porcine COCs isolated from antral ovarian follicles (FIG. 3 E; see also FIG. 6 A, B).

A distinct, dense layer of ubiquitin-cross-reactive proteins was detected in the outer layer of porcine zona in the oocytes from both preantral and antral follicles on both ovarian tissue sections (FIG. 3 A-D, E, F), and on the whole mount preparations (FIG. 3 E, FIG. 6 A, B O). The most striking feature of these (ubiquitin-immuno-reactive) proteins was the fact that they were detected exclusively on the outer face of ZP, which is in contrast with the homogeneous distribution of major ZP components, ZP 1, 2 and 3 throughout mammalian zona (Thaler and Cardullo, 2002; Wassarman and Litscher 1995). Similarly, the sugar residues present in zona glycoproteins are either on the inner face of ZP, or dispersed evenly throughout it (Aviles *et al.,* 1997; 2000). This localization of ubiquitin immuno-reactivity was not due to the limited penetration of anti-ubiquitin antibodies through ZP since such data were obtained by surface staining of tissue sections. Furthermore, an unrelated polyclonal anti-ubiquitin antibody AB1690 also recognized this layer of ubiquitinated proteins on the outer face of ZP (FIG. 3 F) and such immuno-reactivity was not found in negative controls combining the omission of KM691 and incubation with preimmune rabbit serum, followed by appropriate fluorescently conjugated immunoglobulins (FIG. 3 G).

Next, it was determined whether some of the ubiquitin-immuno-reactive ZP substrates undergo degradation after sperm binding to ZP, and whether such degradation can be prevented by proteasomal inhibitors. Oocytes were fertilized in presence/absence of MG132, harvested at 6 h p.i. and loaded onto PAGE gels zona-intact (ZI or Proteasomal+) or zona free (ZF or ZP-) at a total number of 350 per lane. Comparison of the ZF (FIG. 4 A, lane 1) and ZI (FIG. 4A, lane 2), fertilized oocytes at 6 h p.i. showed a number of ubiquitin-immuno-reactive bands which were contributed by the ZP. Interestingly, a set of lanes migrating slightly below the 123 kDa marker were absent from the ZP+/MG132- control oocytes, but not from the ZP+ MG132+ oocytes (FIG. 4 A, lane 3). Such bands were not present in control lysates of human (FIG. 4A, lane 4) and boar (FIG. 4 A, lane 5) spermatozoa. Surprisingly, the Coomassie blue staining (FIG. 4 A; right panel) of the oocyte gels after protein transfer showed that some of such bands were not transferred completely from gels to membranes. Similarly to ubiquitin blot, such bands were completely absent from the ZF oocyte-lane and dramatically reduced in the ZP+/MG132-lane. Large amount of such proteins present in the ZP+/MG132+ suggests that such proteins are digested during control fertilization in the absence of MG132 and that this degradation is effectively prevented by the addition of MG132 into fertilization medium. It is not clear why some of such proteins transfer poorly, though high lysine, a hydrophilic AA residue content of proteins such as poly-ubiquitin could contribute to such effect.

The above observations directed attention to the total protein content of zygotes, assessed by Coomassie blue staining of gels prior to protein transfer (FIG. 4 B). Similar to gels and membranes shown in FIG. 4 A, a set of bands migrating below 123 kDa was dramatically reduced in the ZP+/MG132- zygotes (lane 2), predominant in the ZP+/MG132+ zygotes (lane 3), and absent from the ZP-/MG132+ (lane 4) zygotes, ZP-/MG132- zygotes (not shown), and from the control sperm extracts (lanes 4 and 5).

In subsequent repeats (FIG. 4 C; left panel), the proteins from ZP+ unfertilized oocytes were resolved and transferred (lane 2), ZP+/MG132- zygotes (lane 3), ZP+/MG132+ oocytes (lane 4) and human (Lane 5) and boar (lane 6) spermatozoa. The membranes were probed with anti-ubiquitin KM-691 (FIG. 4 C; left), stained the gel with Coomasie blue after protein transfer (FIG. 4 C; right) and overlapped the gel with the anti-ubiquitin-probed membrane (FIG. 4 C; center). Again, the transfer of a set of bands below the 123 kDa marker was hindered, with a single band present in the ZP+/MG132+ lane, and lesser density, lower MW bands in the M-II and ZP+/MG132- lanes. On the blotted membrane, a new ubiquitin-immuno-reactive band of approximately 30 kDa (FIG. 3D, arrows) appeared in addition to a ladder of lesser density bands (arrows) in the ZP+/MG132- zygotes, but not in the M-II oocytes or in the ZP+MG132+ zygotes. It is thus possible that this band was a product of protein degradation that occurred after fertilization and was prevented by MG132. Similar to previous trial, the set of bands below 123 kDa marker was reduced in the fertilized ZP+/MG132- control zygotes, but not in the M-II oocyte and in the ZP+/MG132+ zygotes (FIG. 4 E). Finally, there was a slight, but discernible reduction in the density of two bands at around 179 kDa level after IVF in the absence of MG132 (FIG. 4 F; arrow). In total, four trials were performed with similar results. The ZP+/MG132+ and ZP+MG132- zygotes were included in all trials, control oocyte lanes varied (M-II/ZP+; M-II/ZP-; IVF/ZP-/MG132+; IVF/ZP-/MG132-). Sperm-oocyte binding was examined by light microscopy in each batch of zygotes used for western blotting and was apparently normal, not affected by MG132-treatment in the ZP+/MG132+ zygotes. Thus, the degradation of several ZP-contributed oocyte proteins occurs after IVF and is prevented by the selective proteasomal inhibitor MG132.

To ascertain that the fertilizing capability of boar spermatozoa was not diminished by some non-specific effect of proteasomal inhibitors on the sperm motility or sperm ability to fuse with the oocyte plasma membrane, porcine oocytes were stripped of ZP and fertilized with acrosome-intact spermatozoa in the presence or absence of MG132 (FIG. 5 A-H). As expected, MG132 inhibited fertilization in the zona-intact oocytes, but had no effect on the zona-free oocytes, 92-96% of which were fertilized, most of them in a polyspermic fashion (FIG. 5 A-H). MG132 had no effect on the fertilization of zona-intact oocytes when either oocytes or spermatozoa were pre-incubated with it and used for insemination after extensive washing. Collectively, these data provide convincing evidence that the proteasomal inhibitors block zona penetration by boar spermatozoa without affecting sperm motility, sperm-zona binding and acrosomal exocytosis.

It was next attempted to visualize the interaction of sperm-acrosomal proteasomes with the ubiquitin immuno-reactive ZP proteins by immunofluorescence combined with differential interference contrast microscopy (DIC) imaging of zygotes 6 h p.i. A mesh-like pattern of ubiquitin labeling was seen on the ZP surface of fertilized zygotes, with reduced fluorescence underneath and around the ZP-bound spermatozoa (FIG. 6 A). In a lateral view (FIG. 6 B-B"), the ubiquitin-immuno-reactive layer under the ZP-bound spermatozoa appeared to be digested (FIG. 6 B'; arrows), producing a vault under the sperm head, clearly discernible under DIC optics (FIG. 6 B"; arrows). Proteasomal subunits MECL-1, α/β-type subunits and β1i, previously detected in the intact sperm acrosome (FIG. 1) were also found in the ZP-bound spermatozoa with the intact acrosomes (Fig 6 C, E, F; arrows), in the acrosomes undergoing exocytosis (FIG. 6 C, F, G; arrowheads) and in the rejected acrosomal shrouds (FIG. 6 C, E; arrowheads). Consistent with the presence of proteasomal subunits and activity of the ubiquitin system in the somatic cell nucleus (Chen *et al.,* 2002; Rivett, 1998), proteasomal subunits of α-type and β-type were detected in the pronuclei inside the fertilized zygotes (FIG. 6 D).

The same proteasomal subunits were detected by colloidal gold TEM in the acrosomal matrix (FIG. 7 A-C) and on the inner acrosomal membrane (FIG. 7 D-G) of the ZP-bound spermatozoa undergoing acrosomal exocytosis and vesiculation (ultrastructure shown in FIG. 7 I-K). As a positive control, the anti-c-Yes antibody (se FIG. 1 A, B for immunofluorescence) recognized mainly the complex of the outer acrosomal membrane and outer-acrosomal perinuclear theca (FIG. 7 H).

### EXAMPLE 5

### PROTEASOMAL INHIBITOR MG132 AND ANTI-PROTEASOMAL SERUM MECL1 INHIBIT BOVINE IN VITRO FERTILIZATION.

The effect of proteasomal interference on the ability of bull spermatozoa to fertilize bovine ova was analyzed *in vitro.* Specific, fully reversible proteasomal inhibitor MG132 (100 µM) was added to fertilization medium at the time of insemination. This allowed fertilization to occur only in 3 out of 103 ova examined at 20 h post insemination (2.8% rate of fertilization; Table 2). The analysis showed that proteasomal interference blocked bovine fertilization partially when 10 µM MG132 was used, but completely when 100 µM Mg132 was applied. Addition of an appropriate amount of ethanol, a vehicle for MG132, had no effect on fertilization or pronuclear development, and neither did the preincubation of unfertilized ova with 100 µM MG132 for 2 h prior to IVF in absence of MG132. Similarly, immune serum against proteasomal subunit MECL1, but not the appropriate preimmune serum, blocked bovine IVF. Proteasomes in pronuclei were detected with an antibody against α-type and β-type proteasomal subunits. DNA was visualized with DAPI (blue).

The ova inseminated in the presence of appropriate amount of 100% ethanol, a vehicle solution for MG132, showed 85.9% fertilization rate. In contrast to irreversible proteasomal inhibitors such as lactacystin, MG132 does not bind to the proteasomal core permanently. However, several groups of ova were preincubated with 100 µM MG132 for 2 hours prior to fertilization to rule out that MG132 could block or hinder fertilization by a direct effect on zona pellucida oolemma or ooplasm, as opposed to an inhibitory effect on sperm acrosome-borne proteasomes (Sutovsky *et al.,* 2003a, b). A 97.5% fertilization rate was observed in such preincubated ova, fertilized in absence of MG132 (Table 2). Similar to the above studies of porcine IVF, rabbit serum against 20S proteasomal core subunit MECL1 effectively blocked *in vitro* fertilization of bovine ova at dilutions of 1/200, 1/500 and even 1/1000 (15.4%, 3.7% and 32.1% fertilization rates, respectively; Table 1). Such effect was not elicited by preimmune rabbit serum at comparable dilutions (Table 1).

Neither MG132 nor anti- MECL1 treatments were accompanied by identifiable anomalies in oocyte morphology. This was identified by immuno-localization carried out on proteasomal subunits MECL1, LMP-2, and α-type and β-type subunits in bull spermatozoa. A negative control was carried out using anti-MECL1 antibody immuno-saturated with appropriate synthetic peptide. Differential interference contrast images were prepared of the same cells. The above observation was consistent with the localization and function identified for spermacrosmal proteasomes in boar above, as well as with the immuno-localization of proteasomal subunits of MECL 1, LMP 2 and α/β in bull spermatozoa.

**Table 2. Proteasomal inhibitor MG132 and anti-proteasomal serum MECL1 inhibit bovine in vitro fertilization and pronuclear development.**

| | **M-II/partheno** | **Partheno** | **Fertilized** | **Total eggs** |
|---|---|---|---|---|
| **A-MECL1 1/200** | 42 | 2 | 8 (15.38%) | 52 |
| **B-MECL1 1/500** | 24 | 2 | 1 (3.7%) | 27 |
| **C-MECL1 1/1000** | 35 | 3 | 18 (32.14%) | 56 |
| **D-SERUM 1/200** | 17 | 1 | 13 (41.94%) | 31 |
| **E-SERUM 1/500** | 15 | 0 | 23 (60.53) | 38 |
| **F-no serum** | 7 | 0 | 19 (73.08%) | 26 |
| **G-MG 100** µ**M 132 at fertilization** | 40 | 0 | 1 (2.44%) | 41 |
| **H-MG 132 100** µ**M ovum preincubatio n2h*** | 1 | 0 | 40 (97.56%) | 41 |
| **I-Ctrl- ethanol at fertilization** | 1 | 1 | 61 (96.82%) | 63 |
| **Total ova** | 182 | 9 | 184 | 375 |

### EXAMPLE 6

### PROTEASOMES INFILTRATE PRONUCLEI FROM THE ONSET OF ZYGOTIC DEVELOPMENT

The ubiquitin-proteasome pathway was until recently ascribed solely to cytoplasmic compartment. The presence and proteolytic activity of proteasomes in the nuclear compartment of somatic cells was analyzed. Specific immune sera were used for the imunolocalization of proteasomal subunits LMP2, α/β and MECL1 in bovine pronuclear zygotes at various stages of pronuclear development. Early after ation into ooplasm, the fully condensed sperm nuclei were shown to be free of detectable proteasomes. Proteasomal subunits started to infiltrate both male and female pronuclei at the initial stage of sperm nuclear decondensation. Both the male and the female pronuclei become occupied by proteasomes prior to reaching full size and apposition. High density of proteasomes was observed inside the apposed, full-sized pronuclei in both monospermic and polyspermic ova, and in cases of asynchronous pronuclear development. Preincubation of ova with 100 µM MG132 prior to fertilization had no effect on the import of proteasomal subunits into pronuclei, and no association of proteasomes with female chromosomes was observed in the ova that remained unfertilized 20 hours after concomitant addition of spermatozoa and 100 µM MG132 into fertilization medium. Negative control using anti-MECL1 antibody saturated with synthetic MECL1 peptide did not show any signal. The sperm tail was visualized by the preincubation of spermatozoa prior to fertilization with a vital mitochondrial dye MitoTracker Green FM (green).

The expression of the above proteasomal subunits in bovine ooplasm was confirmed by Western blotting. Proteasomal subunit MECL1 was detected in lysates of bovine ova (FIG. 8, lane 1), bull spermatozoa (FIG. 8, lane 2) and bovine cumulus cells (FIG. 8, lane 3). In addition to the expected 29 kDa lane in oocyte lysates, higher MW lanes, commonly seen in cell lysates of various cell types (Groettrup *et al.,* 1996) were present in sperm and cumulus cell lysates, probably as a result of incomplete dissociation of MECL1 subunit from other proteasomal subunits.

### EXAMPLE 7

### PROTEASOMAL INTERFERENCE PREVENTS NORMAL PRONUCLEAR DEVELOPMENT AND CAUSES PREMATURE CHROMOSOME CONDENSATION IN FERTILIZED OVA

In the course of studies aimed to determine optimal concentrations of MG132 for the inhibition of bovine fertilization, it was observed that at a low concentration (10 µM), MG132 treatment inhibited the fertilization only partially. The average fertilization rate from two separate trials was 38.2% (n=55) with 10 µM MG132 and 75.7% in control (n=37). Such fertilization in presence of low concentration MG132 allowed the observation of the effects of proteasomal interference on pronuclear development. Pronuclei in the fertilized ova were visualized by the combination of monoclonal antibody mAb 414 recognizing a group nuclear pore complex (NPC) proteins including, Nup 153 and other, related nucleoporins. DNA was stained with blue fluorecent DAPI and sperm tails inside the fertilized ova were identified by the presence of green fluorescence, which accumulated in sperm mitochondrial sheath during sperm preincubation with MitoTracker GreenFM. Nucleoporin labeling was chosen because it is an appropriate marker of nuclear envelope integrity and functionality throughout early stages of pronuclear/zygotic development in bovine (FIG. 12A; Sutovsky *et al.,* 1998) and murine (FitzHarris *et al.,* 2003) zygotes.

Abnormal pronuclear development and premature chromosome condensation (PCC) was observed in bovine ova fertilized in presence of 10 µM MG132, a condition partially permissive to sperm penetration. Pronuclei were visualized by the combination of monoclonal antibody against nuclear pore complex (NPC; red) protein, nucleoporin Nup 153, and the DNA stain DAPI (blue). Invariably, the patterns of PN development in such ova were abnormal. Combination of premature chromosome condensation (PCC) and failed pronuclear apposition was the most common abnormality in both monospermic and polyspermic ova. In some cases, an incomplete decondensation of sperm nucleus was observed, accompanied by PCC of female chromatin. The scattering of chromosomes in PCC and the assembly of presumably soluble nucleoporins around the condensed chromatin were observed. Such pool of nucleoporins was likely not derived from a paternal contribution: Bull spermatozoa carry only a residual amount of nucleoporins (Sutovsky *et al.,* 1999) located in the redundant nuclear envelope (Ho and Suarez, 2003) of the sperm tail connecting piece.

### EXAMPLE 8

### ANALYSIS OF MECHANISM OF PREVENTION OF NORMAL PRONUCLEAR DEVELOPMENT

In previous studies, NPC and annulate lamellae-like arrangements of nucleoporins were occasionally observed in aged metaphase-II ova progressing towards spontaneous activation. However, it should be considered that the sperm nuclei exposed to MG132 treated cytoplasm also underwent premature chromosome condensation. This suggests that proteasomal degradation may not be necessary for the initial decondensation of the sperm chromatin, but is necessary for full development of the male pronucleus. Such effect could be direct via protesome-dependent remodeling of maternal and paternal chromatin inside the zygote or indirect such as the block of proteasome-dependent cell cycle progression. The progression of meiosis and pronuclear development appear to be inhibited by MG132 in rat (Josefsberg *et al.,* 2000). This observation was put in use for creating the first cloned rat offspring, wherein the investigators applied MG132 to prevent premature activation of rat ova (Zhou *et al.,* 2003).

The failure of PN apposition could be due to the absence of intact nuclear envelope, to which sperm-aster microtubules are anchored during pronuclear apposition, or to a block of proteasomal function in the sperm tail connecting piece, from which the sperm centriole must be liberated prior to zygotic centrosome reconstitution and sperm aster formation (Sutovsky *et al.,* 1996; Sutovsky and Schatten, 1997; Wojcik *et al.,* 2000).

### EXAMPLE 9

### REDUCTION OF POLYSPERMY DURING PORCINE IN VITRO FERTILIZATION TO PRODUCE BALSTOCYSTS FOR EMBRYO-TRANSFER

Proteasomal interference may be used to alleviate or reduce the rate of polyspermic fertilization during IVF without reducing overall rate of fertilization. Subsequently, new additives to fertilization media can be formulated that will allow to increase the production of normal, diploid blastocysts for embryo transfer programs by reducing the number of non-viable polyploid blastocysts derived from polyspermic zygotes. In intial studies (Table 3) the penetration of ZP was completely inhibited by specific proteasomal inhibitors MG-132 and lactacystin at 100 uM concentration. At a threshold concentration of 10 uM, proteasomal inhibitor lactacystin did not prevent fertilization, but reduced the rates of polyspermic fertilization to 10%, while increasing the rate of monospermic fertilization to 65%. Control fertilization in this study had polyspermy and monospermy rates of 44 and 38%, respectively, as evaluated by the number of fluorescently labeled sperm tails inside the zygotes at 20 h p.i..

**Table 3: Reduction of polyspermy during porcine IVF by the addition of 10 uM lactacystin into culture medium at the time of insemination.**

| | **10 uM Lactacystin** | **10 uM MG 132** | **Control** |
|---|---|---|---|
| **Polyspermic** | 2 (10%) | 0 | 7 (44%) |
| **Monospermic** | 13 (65%) | 0 | 6 (38%) |
| **Not fertilized** | 5 (25%) | 19 (100%) | 3 (18%) |
| **Total** | 20 | 19 | 16 |

### Preferred Embodiments of the Present Invention are as Follows:

1. A method of contraception comprising inhibiting proteasomal activity of spermatozoa.
2. The method of embodiment 1, wherein proteasomal activity of spermatozoa is inhibited *in vitro*.
3. The method of embodiment 1, wherein proteasomal activity of spermatozoa is inhibited *in vivo*.
4. The method of embodiment 1, wherein proteasomal activity is inhibited by antibodies that bind and inhibit proteasome activity.
5. The method of embodiment 4, wherein the antibodies comprise humanized antibodies.
6. The method of embodiment 1, wherein proteasomal activity is inhibited by passive immunization with an antibody.
7. The method of embodiment 4, wherein the antibodies are induced by immunization of a subject with at least one antigen of a proteasomal peptide.
8. The method of embodiment 7, wherein the antigen is an antigenic proteasomal peptide.
9. The method of embodiment 8, wherein the at least one antigenic peptide is comprised in a vaccine.
10. The method of embodiment 9, wherein the one or more antigenic proteasomal peptide comprises an amino acid sequence of SEQ ID NO:1 or SEQ ID NO:2.
11. The method of embodiment 7, wherein the antigenic proteasomal peptide is a fusion protein.
12. The method of embodiment 7, wherein multiple antigenic epitopes are present in a single protein.
13. The method of embodiment 4, wherein antibodies are induced by immunization with at least one polynucleotide encoding at least one antigenic proteasomal peptide.
14. The method of embodiment 13, wherein the polynucleotide encoding at least one antigenic proteasomal peptide is comprised in an expression vector.
15. The method of embodiment 14, wherein the expression vector is a plasmid expression vector.
16. The method of embodiment 1, wherein proteasome activity is inhibited by contacting spermatozoa with a proteasome inhibitor.
17. The method of embodiment 16, wherein the proteasome inhibitor is comprised in a pharmaceutically acceptable formulation.
18. The method of embodiment 16, wherein the proteasome inhibitor is MG132 or lactacystin.
19. The method of embodiment 17, wherein the pharmaceutically acceptable formulation is formulated into solutions, suspensions, tablets, pills, capsules, sustained release formulations, powders, creams, ointments, salves, sprays, pumps, liposomes, suppositories, inhalants, or patches.
20. The method of embodiment 17, wherein a route of administration of a pharmaceutically acceptable formulation is intravenous, intraperitoneal, intradermal, intramuscular, intrauterine, dermal, nasal, buccal, vaginal, inhalation, or topical.
21. The method of embodiment 20, wherein the route of administration is intrauterine.
22. A method for inducing anti-proteasome antibodies in a mammal, the method comprising administering to the mammal one or more proteasomal peptides, wherein the administering induces production of an antibody that binds and inhibits the activity of a mammalian proteasome.
23. The method of embodiment 22, wherein the mammal is a human.
24. The method of embodiment 22, wherein one or more proteasomal peptides are administered by expression of a polynucleotide encoding a proteasomal peptide.
25. The method of embodiment 24, wherein the polynucleotide encoding the proteasomal peptide is administered by intramuscular, intravenous, subcutaneous, intraperitoneal, intradermal, oral or inhaled route of administration.
26. The method of embodiment 22, wherein the proteasomal peptide is administered by intramuscular, intravenous, subcutaneous, intraperitoneal, intradermal, oral or inhaled route of administration.
27. A method of contraception comprising inhibiting the degradation of ubiquitin associated with an oocyte.
28. The method of embodiment 27, wherein in inhibition of ubiquitin degradation is by administration of an antibody against ubiquitin, ubiquitin-activating enzyme, ubiquitin-carrier/conjugating enzyme or ubiquitin-ligase.
29. A method of evaluating fertility comprising detecting the presence or absence of proteins associated with ubiquitin-proteasome pathway in or on spermatozoa.
30. The method of embodiment 29, wherein detecting the presence or absence of ubiquitin-proteasome associated proteins is by detecting binding of antibodies or peptides that bind specifically to ubiquitin-proteasome associated proteins.
31. The method of embodiment 30, wherein detecting is by immunofluorescence or flow cytometry.
32. A method of *in vitro* fertilization comprising adding to a sperm sample used for said *in vitro* fertilization an inhibitor of proteasomal activity, wherein the inhibitor is added in a concentration insufficient to prevent fertilization but sufficient to prevent polyspermic fertilization.
33. The method of embodiment 32, wherein less than about 25% of fertilizations are polyspermic.
34. The method of embodiment 32, wherein less than 10% of fertilizations are polyspermic.
35. The method of embodiment 32, wherein the inhibitor is added *in vitro.*
36. The method of embodiment 32, wherein the inhibitor is administered *in vivo.*

### References

U.S. Patents 4,148,876, 4,179,337, 4,406,885, 4,444,887, 4,512,972, 4,554,101, 4,659,774, 4,682,195, 4,683,202, 4,710,111, 4,816,397, 4,816,567, 4,816,571, 4826,687, 4,883,750, 4,959,463, 5,141,813, 5,225,539, 5,264,566, 5,413,923, 5,428,148, 5,429,599, 5,484,719, 5,530,101, 5,545,806, 5,554,744, 5,565,203, 5,565,332, 5,569,825, 5,574,146, 5,585,089, 5,589,466, 5,593,972, 5,602,244, 5,612,487, 5,614,610, 5,614,610, 5,625,126, 5,633,425, 5,645,897, 5,661,016, 5,670,488, 5,707,644, 5,766,588, 5,766,588, 5,776,451, 5,807,715, 5,814,295, 5,814,318, 5,830,725, 5,851,826, 5,869,451, 5,871,982, 5,879,934, 5,885,793, 5,888,502, 5,910,306, 5,914,123, 5,916,771, 5,933,819, 5,939,598, 5,942,242, 5,955,331, 5,980,898, 5,985,318, 6,004,807, 6,034,298, 6,136,320, 6,194,560, 6,210,963, 6,248,565, 6,287,571, 6,328,958, 6,339,086, 6,348,449, 6,348,450, 6,359,054, 6,383,795
Aviles et al., Biol. Reprod., 57(5):1155-1163, 1997.
Aviles et al., Mol. Reprod. Dev., 57(3):296-308, 2000.
Balicki and Beutler, Medicine, 81(1):69-86, 2002.
Bangham et al., J. Mol. Biol., 13(1):253-259, 1965.
Barany and Merrifield, In: The Peptides, Gross and Meienhofer (eds.), Academic Press, NY, 1-284, 1979.
Bialy et al., Folia Hostochem. Cytobiol., 39:129-130, 2001.
Bilbao et al., FASEB J., 11(8):624-634, 1997.
Bogyo et al., Biopolymers Peptide Science, 43(4):269-280, 1997.
Caplen et al., Gene Ther., 6(3):454-459, 1999.
Chen et al., Nature, 415:545-549, 2002.
Coffey et al., Science, 282(5392):1332-1334, 1998.
Conaway et al., Science, 296(5571):1254-1258, 2002.
Deamer and Uster, In: Liposome Preparation: Methods and Mechanisms, Lipsomes, Ostro (Ed.), 1983.
Einspanier et al., J. Reprod. Fertil., 98(1):241-244, 1993.
EPs 0 598 877, 0 239,400, 0 519,596, 0 592,106, 0 320 308
Evans, Bioessays, 23(7):628-639, 2001.
Fairlie et al., Curr. Med. Chem., 5(1):29-62, 1998.
Feng et al., Nat Biotechnol, 15(9):866-870, 1997.
Fenteany et al., Science, 268(5211):726-731, 1995.
Fisher et al., Hum. Gene Ther., 7(17):2079-2087, 1996.
FitzHarris et al., Mol. Biol. Cell, 14(1): 288-301, 2003.
Gerton, In: Fertilization, Hardy (Ed.), San Diego, Academic Press, 265-302, 2002.
Gillies et al., J. Immunol. Methods, 125(1-2):191-202, 1989.
Glenn et al., Invest. Drugs, 8:797-805, 1999.
Glickman and Ciechanover, Physiol. Rev., 82(2):373-428, 2002.
Gnant et al., Cancer Res., 59(14):3396-403, 1999a.
Gnant et al., J. Natl. Cancer Inst" 91(20):1744-1750, 1999b.
Goldberg et al., Chem. Biol., 2:503-508, 1995.
Gregoriadis, DRUG CARRIERS IN BIOLOGYAND MEDICINE, Gregoriadis (ed.), 287-341, 1979.
Groettrup et al., Nature, 381(6578):166-168, 1996.
Hayashi et al., J. Immunol., 159(6): 2760-2770, 1997.
Hermo and Jacks, Mol. Reprod. Dev., 63(3):394-410, 2002.
Hershko and Ciechanover, Annu. Rev. Biochem., 67:425-479, 1998.
Ho and Suarez, Biol. Reprod., 68(5):1590-1596, 2003.
Hochstrasser, Mol. Cell, 9(3):453-454, 2002.
Holzer et al. Virology, 253(1):107-114, 1999.
Hosaka et al., J. Virol., 46(3):1014-1017, 1983.
Huang et al., Virology, 97:212-217, 1979.
Imai et al., Nephrologie, 19(7):397-402, 1998.
Josefsberg et al., Biol. Reprod., 62(5): 1270-1277, 2000.
Kaneda, Adv Drug Deliv. Rev., 43(2-3):197-205, 2000.
Klein et al., Nature, 327:70, 1987.
Kohler et al., Virology, 289(2):186-191, 2001.
Kyte and Doolittle, J Mol Biol, 157(1):105-32, 1982.
Laney and Hochstrasser, Cell, 97:427-330, 1999.
Leclerc and Goupil, Biol. Reprod., 67(1):301-307, 2002.
Lee and Goldberg, Trends Cell Biol., 8(10): 397-403, 1998.
Lippert et al., J. Androl., 14(2):130-131, 1993.
Liu and Huang. J. Control Release, 78(1-3):259-66, 2002.
Lonberg and Huszar, Int. Rev. Immunol., 13: 65-93, 1995.
Lundstrom, J. Recept. Signal Transduct. Res., 19(1-4):673-686, 1999.
Matsumura and Aketa, Mol. Reprod. Dev., 29:189-119, 1991.
McCauley et al., Biol. Reprod., 66(1):311, 2002.
Merrifield, Science, 232: 341-347, 1986.
Moore, Trends Pharmacol Sci., 15(4):124-129, 1994.
Morrison, Science, 229(4719):1202-1207, 1985.
Mullis et al., Biotechnology, 24:17-27, 1992.
Mullis, Sci. Am., 262(4):56-61, 64-65, 1990.
Neumann et al., Proc. Natl. Acad. Sci. USA, 96(16):9345-9350, 1999.
Nicolau et al., Methods Enzymol., 149:157-176, 1987.
O'Reilley et al., In: Baculovirus Expression Vectors, A Laboratory Manual, Oxford University Press, 1994.
Ostrowska et al., Int. J. Biochem. Cell Biol., 32(7):747-757, 2000.
Padlan, Mol. Immunol., 28(4-5):489-498, 1991.
Pickart, In: Ubiquitin and the biology of the cell, Peters et al., (Eds.), Plenum Press, NY, 19-63, 1998.
Primakoff and Myles, Science, 296:2183-2185, 2002.
Ravid et al., In: DNA Transfer to Cultured Cells, Wiley and Sons, 1998.
Rivett, Curr. Opin. Immunol., 10(1): 110-114, 1998.
Robbins and Ghivizzani, Pharmacol Ther, 80(1):35-47, 1998.
Robbins et al., Trends Biotech.., 16(1):35-40, 1998.
Roguska et al., Proc. Natl. Acad. Sci. USA, 91(3):969-973, 1994.
Sambrook et al., In: Molecular cloning, Cold Spring Harbor Laboratory Press, Cold Spring Harbor, NY, 2001.
Sanford et al., Technique J. Methods Cell Molec. Biol., 3:3-16, 1991.
Sawada et al., Mol. Reprod. Dev., 62:271-276, 2002a.
Sawada et al., Proc. Natl. Acad. Sci. USA, 99:1223-1228, 2002b.
Schmid and Briand, Mol. Biol. Reports, 24(1-2):1-138, 1997.
Stewart and Young, Solid Phase Peptide Synthesis, 2d. ed., Pierce Chemical Co. 1984.
Studnicka et al., Protein Eng., 7(6):805-814, 1994.
Sutovsky and Schatten, Biol. Reprod., 56(6): 1503-1512, 1997.
Sutovsky et al., Biol. Reprod., 55(6):1195-1205, 1996.
Sutovsky et al., Biol. Reprod., 68(5): 1793-1800, 2003.
Sutovsky et al., J. Cell Sci., 111(Pt 19):2841-2854, 1998.
Sutovsky et al., J. Cell Sci., 114(Pt 9):1665-1675, 2001a.
Sutovsky et al., Human Reprod., 16:250-258, 2001b.
Sutovsky et al., Mol. Reprod. Dev., 61(3): 406-413, 2002.
Sutovsky et al., Nature, 402(6760):371-372, 1999.
Sykes and Johnston, Nature Biotechnology, 17:355-359, 1999.
Szoka and Papahadjopoulos, Proc. Natl. Acad. Sci. USA, 75:4194-98, 1978.
Talbot et al., Biol. Reprod., 68(1):1-9, 2003.
Tam et al., J. Am. Chem. Soc., 105:6442, 1983.
Tanaka and Tsurumi, Mol. Biol. Rep., 24(1-2):3-11, 1997.
Thaler and Cardullo, Biol. Reprod., 66(1):65-69, 2002.
Thompson et al., J. Reprod. Fertil., 109(2):337-348, 1997.
Voges et al., Annu. Rev. Biochem., 68:1015-1068, 1999.
Wassarman and Litscher, Curr. Top Dev. Biol., 30:1-19, 1995.
Watt et al., Proc. Natl Acad. Sci. USA, 83(2):3166-3170, 1986.
WOs 91/09967, 91/10741, 92/01047, 96/33735, 96/33735, 96/34096, 96/34096, 98/16654, 98/24893, 98/24893, 98/46645, and 99/50433
Wojcik et al., Int. J. Androl., 23(3): 169-177, 2000.
Wu and Wallace, Genomics, 4(4):560-569, 1989.
Zhou et al., Science, 302(5648):1179, 2003.

## Claims

1. Use of an antibody, in particular of a humanized antibody, that binds to an antigenic proteasomal peptide for contraception in a mammal.

2. The use of claim 1, wherein the antigenic proteasomal peptide comprises a proteasomal subunit, in particular wherein the antigenic proteasomal peptide is a fusion protein.

3. Use of at least one antigenic proteasomal peptide for contraception in a mammal.

4. The use of claim 3, wherein the antigenic proteasomal peptide comprises a proteasomal subunit or is a fusion protein.

5. The use of claim 3, wherein use of the antigenic proteasomal peptide induces the production of an antibody that binds to at least one antigenic proteasomal peptide.

6. The use of claim 3, wherein the antigenic peptide is comprised in a vaccine.

7. Use of at least one polynucleotide encoding at least one antigenic proteasomal peptide for contraception in a mammal.

8. The use of claim 7, wherein the polynucleotide is comprised in an expression vector, in particular wherein the expression vector is a plasmid expression vector.

9. A method of preparing a composition for contraception in a mammal by formulating at least one antigenic proteasomal peptide into the composition.

10. The method of claim 9, wherein the at least one antigenic proteasomal peptide is derived from a proteasomal subunit.

11. The method of claim 9, wherein the at least one antigenic proteasomal peptide induces the production of an antibody that binds to at least one antigenic proteasomal peptide. or wherein the antigenic peptide is comprised in a vaccine.

12. A method of preparing a composition for contraception in a mammal by formulating an antibody, in particular a humanized antibody, that is immunoreactive with a component of the proteasome pathway into the composition.

13. The method of claim 12, wherein the component of the proteasome pathway is a proteasomal peptide or polypeptide, in particular wherein the proteasomal peptide is derived from a proteasomal subunit or is in the context of a fusion protein.

14. A method of preparing a composition for contraception in a mammal by formulating at least one polynucleotide encoding at least one antigenic proteasomal peptide into the composition.

15. The method of claim 14, wherein the polynucleotide is comprised in an expression vector, in particular wherein the expression vector is a plasmid expression vector.
